# EUROPEAN PATENT APPLICATION

(11) **EP 4 840 514 A2**
(43) Date of publication of application: **18.11.2026**
(21) Application number: 26189655.9
(22) Date of filing: 25.07.2019
(51) Int. Cl.: A61P 35/00

(54) **NOVEL HYALURONIC ACID-HYDROLYZING ENZYME MUTANT AND PHARMACEUTICAL COMPOSITION COMPRISING SAME**

(30) Priority: 25.07.2018 KR 20180086308; 15.03.2019 KR 20190029758
(62) Document number(s) of the earlier application(s) in accordance with Art. 76 EPC: 19827585.1 / 3 636 752
(71) Applicant: Alteogen, Inc, Yuseong-gu Daejeon 34054 (KR)
(72) Inventor: Park, Soon Jae, Daejeon 34054 (KR); Chung, Hye-Shin, Daejeon 34054 (KR); Lee, Seung Joo, Daejeon 34054 (KR); You, Sun-Ah, Daejeon 34054 (KR); Song, Hyung-Nam, Daejeon 34054 (KR); Lee, Chang Woo, Daejeon 34054 (KR)
(74) Representative: Leonard, Thomas Charles

(57) **Abstract**

The present invention is related to the field of protein engineering technology which increases the enzymatic activity and thermal stability of human hyaluronidase which is an enzyme that hydrolyzes hyaluronic acid; and more particularly to hyaluronidase PH20 variants or fragments thereof, which comprise one or more amino acid residue substitutions in the region corresponding to the alpha-helix region and/or its linker region in the amino acid sequence of wild-type PH20 of SEQ ID NO: 1 and in which one or more amino acid residues at the N-terminus and/or the C-terminus are selectively truncated additionally.

Specifically, the present invention relates to PH20 variants or fragments thereof, which comprise one or more amino acid residue substitutions selected from the group consisting of T341A, T341C, T341C, S343E, M345T, K349E, L353A, L354I, N356E and I361T in wild-type PH20 having the amino acid sequence of SEQ ID NO: 1, and additionally comprise the substitution of amino acids located in the alpha-helix 8 region and/or a linker region between alpha-helix 7 and alpha-helix 8 in the amino acid sequence of wild-type PH20, and in which portion(s) of amino acids located at the N-terminal and C-terminal regions are deleted.

## Description

### TECHNICAL FIELD

The present invention relates to novel human hyaluronidase variants having increased enzymatic activity and thermal stability compared to human hyaluronidase which is an enzyme that hydrolyzes hyaluronic acid, and more particularly to hyaluronidase PH20 variants or fragments thereof, which comprise one or more amino acid residue substitutions in the region corresponding to the alpha-helix region and/or its linker region in wild-type PH20 having the amino acid sequence of SEQ ID NO: 1, preferably mature wild-type PH20 consisting of amino acid residues L36 to S490, and in which portion(s) of the N-terminal and/or C-terminal amino acid residues are selectively deleted, a method for producing the same, and a pharmaceutical composition comprising the same.

### BACKGROUND ART

The human skin is composed of epidermis, dermis and a subcutaneous fat layer, and there are six types of glycosaminoglycans in the skin. These glycosaminoglycans include hyaluronic acid, chondroitin sulfate, dermatan sulfate, heparan sulfate, heparin, and keratin sulfate.

These glycosaminoglycans are composed of repeating disaccharide sugar units. The number of the disaccharide sugar units is different among the glycosaminoglycans, but ranges from several hundreds to thousands. Among the glycosaminoglycans, hyaluronic acid is present in the skin more than half of the amount in the body. Hyaluronic acid is synthesized by hyaluronan synthase present in the cell membrane, is present alone without binding to proteoglycans, and is the only glycosaminoglycan having no sulfate group. Other glycosaminoglycans bind to proteoglycans and have a sulfate group. Hyaluronic acid consists of glucuronic acid and N-acetylglucosamine linked via β-1,3 bonds, and is composed of about 5,000 repeating units of these disaccharides. N-acetylglucosamine and glucuronic acid are linked via β-1,4 bonds. It is known that about one-third (5 g) of hyaluronic acid in the human body is turned over every day.

Hyaluronidases are enzymes that degrade hyaluronic acid present in the extracellular matrix. It is known that there are six types of hyaluronidases in humans: They are Hyal1, Hyal2, Hyal3, Hyal4, HyalPS1, and PH20/SPAM1. Human Hyal1 and Hyal2 are expressed in most of the tissues. PH20/SPAM1 (hereinafter referred to as PH20) is expressed in the sperm plasma membrane and the acrosomal membrane. However, HyalPS1 is not expressed because it is a pseudogene. Hyaluronidases are divided into, according to a method of cleaving hyaluronic acid, three types: enzymes (EC 3.2.1.35) that cleave β-1,4 bonds between N-acetylglucosamine and glucuronic acid by the use of H₂O; enzymes (EC 3.2.1.36) that cleave β-1,3 bonds between N-acetylglucosamine and glucuronic acid by the use of H₂O; and bacterial hyaluronidases (EC 4.2.99.1) that cleave β-1,4 bonds without using H₂O.

The catalytic amino acids of Hyal1 are D129 and E131, which hydrolyze hyaluronic acid by substrate-assisted catalysis. Hyal1 exhibits the optimum activity at an acidic pH of 3 to 4, and has no enzymatic activity at pH 4.5 or higher. In contrast to Hyal1, PH20 exhibits enzymatic activity at a wide pH range of 3 to 8.

Arming et al. identified that the catalytic amino acids of PH20 are D111 and E113 (Arming et al., 1997). Arming et al. labelled Leu, the first amino acid of PH20 from which the signal sequence was removed, as position 1, and thus the catalytic amino acid residues of the full-length PH20 included in the signal sequence correspond to D146 and E148, respectively.

Hyaluronidase hydrolyzes hyaluronic acid, thereby reducing the viscosity of hyaluronic acid in the extracellular matrix and increasing the permeability thereof into tissue (skin). The subcutaneous area of the skin has a neutral pH of about 7.0 to 7.5. Thus, among the various types of hyaluronidases, PH20 is widely used in clinical practice (Bookbinder et al., 2006). In examples in which PH20 is used in clinical practice, PH20 is used as an eye relaxant and an anesthetic injection additive in ophthalmic surgery, and is also co-administered with an antibody therapeutic agent which is injected subcutaneously (Bookbinder et al., 2006). In addition, based on the property of hyaluronic acid that is overexpressed in tumor cells, PH20 is used to hydrolyze hyaluronic acid in the extracellular matrix of tumor cells, thereby increasing the access of an anticancer therapeutic agent to the tumor cells. In addition, it is also used to promote resorption of body fluids and blood, which are excessively present in tissue.

PH20 was first identified in guinea pig sperm by Lathrop et al. and is also known to be expressed in sperms of different species. Human PH20 gene was cloned by Lin et al. and Gmachl et al. Human PH20 has the amino acid sequence of SEQ ID NO: 1 which consists of 509 amino acid residues, and exhibits 60% amino acid identity with guinea pig PH20. Human PH20 enzyme is encoded from SPAM1 (sperm adhesion molecule-1) gene, and Ser490 of PH20 is present as binding to the glycosylphosphatidylinositol (GPI) on the surface of the sperm plasma membrane and in the acrosomal membrane. Sperm hydrolyzes hyaluronic acid using PH20 when it penetrates oocytes through the hyaluronan-rich cumulus layer of the oocytes. PH20 is present in the amount corresponding to 1% or less of the amount of proteins in sperm, and has six N-glycosylation sites (N82, N166, N235, N254, N368, and N393).

Currently commercially available PH20 is obtained by extraction from the testes of cattle or sheep. Examples thereof include Amphadase^{®} (bovine hyaluronidase) and Vitrase^{®} (sheep hyaluronidase).

Bovine testicular hyaluronidase (BTH) is obtained by removing a signal peptide and 56 amino acids on the C-terminal from bovine wild-type PH20 during post-translational modification. BTH is also a glycoprotein, and has a mannose content of 5% and a glucosamine content of 2.2%, based on the total components including amino acids. When animal-derived hyaluronidase is repeatedly administered to the human body at a high dose, a neutralizing antibody can be produced. Since animal-derived hyaluronidase contains other biomaterials in addition to PH20, it may cause an allergic reaction when administered to the human body (Bookbinder et al., 2006). In particular, the production and the use of PH20 extracted from cattle can be limited due to concerns of mad cow disease. In order to overcome this problem, studies on the recombinant protein of human PH20 have been conducted.

Recombinant protein of human PH20 has been reported to be expressed in yeast (*P. pastoris*)*,* DS-2 insect cells, and animal cells. The recombinant PH20 proteins produced in insect cells and yeast differ from human PH20 in terms of the pattern of N-glycosylation during post-translational modification.

Among hyaluronidases, protein structures of Hyal1 (PDB ID: 2PE4) (Chao et al., 2007) and bee venom hyaluronidase (PDB ID: 1FCQ, 1FCU, 1FCV) are determined. Hyal1 is composed of two domains, a catalytic domain and an EGF-like domain. The catalytic domain is in the form of (β/α)₈ in which an alpha-helix and a beta-strand, which characterize the secondary structure of the protein, are each repeated eight times (Chao et al., 2007). The EGF-like domain is conserved in all variants in which the C-terminus of Hyal1 is spliced differently. The amino acid sequences of Hyal1 and PH20 are 35.1% identical, and the protein structure of PH20 has not yet been found.

A recombinant protein of human PH20 was developed by Halozyme Therapeutic, Inc. and has been sold under the trade name Hylenex^{®} (Bookbinder et al., 2006; Frost, 2007).

When D146 and E148, which are the catalytic amino acids of PH20, were mutated to asparagine (D146N) and glutamine (E148Q), respectively, there was no enzymatic activity (Arming et al., 1997). In addition, when R246 of PH20 was substituted with glycine, the enzymatic activity was reduced by 90%, and when E319 was substituted with glutamine and R322 was substituted with threonine, the enzymatic activity disappeared. A variant in which 36 amino acids at the C-terminus of PH20 were removed (474-509 amino-acid truncation) showed a 75% reduction in enzymatic activity compared to wild-type PH20. This variant was not secreted extracellularly and remained in HeLa cells. When C-terminal 134 amino acids were removed from PH20, PH20 had no enzymatic activity and was not secreted extracellularly. According to Frost et al., the C-terminal 477-483 region of PH20 is essential for soluble expression (Frost, 2007). The activity of full-length PH20 (1 to 509) or a PH20 variant having a C-terminus truncated at position 467 was merely 10% of a PH20 variant having a C-terminus truncated at one of positions 477 to 483 (Frost, 2007).

Meanwhile, recombinant PH20 still has insufficient thermal stability or expression levels. Therefore, there is a great demand in industry for a recombinant hyaluronidase having further improved characteristics.

### Summary of the Invention

It is an object of the present invention to provide a novel hyaluronidase PH20 variant or fragment thereof which is improved in stability, enzyme activity and expression level, compared to wild-type PH20, preferably mature wild-type PH20.

Another object of the present invention is to provide a composition for treating cancer, comprising the above-described hyaluronidase PH20 variant or fragment thereof, and a method of treating cancer using the same.

To achieve the above objects, the present invention provides a hyaluronidase PH20 variant or fragment thereof, which comprises one or more amino acid residue substitutions in the region corresponding to an alpha-helix region and/or its linker region in the amino acid sequence of wild-type PH20, preferably mature wild-type PH20, and in which portion(s)of the N-terminal and/or C-terminal amino acid residues are selectively deleted.

The present invention also provides a composition for treating cancer, comprising the above-described hyaluronidase PH20 variant or fragment thereof, and a method of treating cancer using the same.

### BRIEF DESCRIPTION OF THE DRAWINGS

FIG. 1 shows the protein tertiary structure model of PH20. The protein tertiary structure of PH20 was modeled using, as a template (Chao et al., 2007), Hyal1 (PDB ID: 2PE4) (Chao et.al., 2007) whose protein crystal structure was found, in Swiss-Model server (https://swissmodel.expasy.org/).
FIG. 1A shows the protein tertiary structure model of PH20, and indicates D146 and E148 which are catalytic amino acids. The protein tertiary structure model of PH20 is composed of eight repeats of a beta-strand and an alpha-helix.
FIG. 1B shows an eta (η) 8 loop in which alpha-helix 8 of PH20 and G340 to I344 which form a linker region in the N-terminal region of alpha-helix 8 are located. G340, T341, L342, S343 and I344 residues are each shown.
FIG. 1C shows amino acid residues (C351, Y357, and N363) which interact with the adjacent secondary structure, among the amino acids located in alpha-helix 8 of PH20. C351 forms a disulfide bond with C60 located in alpha-helix 1, Y357 hydrophobically interacts with F315 located between beta-strand 7 and alpha-helix 7, and N363 forms a hydrogen bond with a D69 residue located in alpha-helix 1.
FIG. 2 compares the protein expression levels of WT (wild type) PH20 and variants constructed in the present invention. WT and variants were expressed by transient transfection in ExpiCHO cells. WT had an expression level of 16.1 mg/L. The protein expression levels of variants based on the variants HM1 and HM6 were higher than that of WT, and the protein expression levels of HM4 and HM7 were the highest. The protein expression level of HM11 obtained by introducing additional amino acid substitutions (Y365F and I367L) into the variant HM6 decreased to 6.4 mg/mL.
FIG. 3 shows experimental results for the variants HM1 and HM6.
FIG. 3A shows the results of SDS-PAGE after purification of WT and the variants HM1 and HM6. Purification was performed using a HisTrap column and a Q Sepharose column. WT and the variants HM1 and HM6 had a molecular weight of approximately 70 kDa (figure legend: M, molecular weight marker; CS, supernatant; FT, flow-through; and Elution, elution fractions).
FIG. 3B shows the enzymatic activity values of WT and the variants HM1 and HM6, measured by turbidimetric assay at pH 7.0. In the present invention, the enzymatic activity value measured by the turbidimetric assay was expressed as specific activity.
FIG. 3C shows the enzymatic activities of WT and the variants HM1 and HM6, measured by substrate-gel assay. After SDS was removed with 2.5% Triton X-100 (w/v) at 4°C, an enzymatic reaction was performed at 37°C for 1 to 4 h. The variant HM6 renatured faster than WT and the variant HM1 and hydrolyzed hyaluronic acid on polyacrylamide gel. The white band shows hyaluronic acid degraded by WT and the variant proteins.
FIG. 3D shows the enzymatic activities of WT and the variant HM1, measured by substrate-gel assay at pH of 5 to 8. WT and the variant HM1 exhibit activity in the pH range of 5 to 8, and show the highest enzymatic activity at pH 5.0. The variant HM1 has the signal peptide of human serum albumin or human Hyal1. The white band shows hyaluronic acid degraded by WT and the variant protein.
FIG. 3E shows the results of separating WT and the variants HM1 and HM6 by a phenyl column. The variants were eluted faster than WT from the phenyl column.
FIG. 4 shows the results of analyzing the final product of hyaluronic acid, degraded by WT and the variant HM6, after 10 min and 1 h by means of an Amide-80 column.
FIG. 5 shows experimental results for G340 to I344 amino acid mutations of PH20.
FIG. 5A shows SDS-PAGE results after HisTrap column purification for variants HM7, HM8, HM9, HM10 and HM21.
FIG. 5B shows the results of measuring the enzymatic activities of WT and variants HM6, HM8, HM9, HM10, HM21 and HM7 by turbidimetric assay at pH 7.0.
FIG. 5C shows the results of measuring the enzymatic activities of WT and variants HM6, HM8, HM9, HM10, HM21 and HM7 by substrate-gel assay. The bar graph at the bottom of FIG. 5C shows the degree of enzymatic activity obtained by quantifying the band after staining the gel with Alcian blue. The white band shows hyaluronic acid degraded by WT and the variant proteins.
FIG. 5D shows the results of analyzing WT and variants HM8, HM9, HM10, HM21 and HM7 by phenyl column chromatography.
FIG. 5E shows the results of separating WT and variants HM6, HM8, HM9, HM10, HM21 and HM7 depending on their isoelectric points at the pH of 3 to 7 by means of IEF gel.
FIG. 6 shows experimental results for the variant HM11.
FIG. 6A shows the results of purifying protein by HisTrap column chromatography for the variant HM11.
FIG. 6B shows the results of measuring the enzymatic activities of WT and the variant HM11 at pH 7.0 by turbidimetric assay.
FIG. 7 shows experimental results for N-terminally truncated PH20 variants HM40, HM13, HM41, HM24, HM42, and HM25.
FIG. 7A shows the results of purifying protein by HisTrap column chromatography for the PH20 variants HM40, HM13, HM41, HM24, HM42 and HM25.
FIG. 7B shows the expression levels of the PH20 variants HM40, HM13, HM41, HM24, HM42, HM25, HP61 and HP62 in ExpiCHO cells.
FIG. 7C shows the enzymatic activities of the PH20 variants HM40, HM13, HM41, HM24, HM42 HM25, HP61 and HP62 measured at pH 7.0 by turbidimetric assay and expressed as specific activities.
FIG. 7D shows the enzymatic activities of the PH20 variants HM40, HM13, HM41, HM24, and HM42, measured by substrate-gel assay. The white band shows hyaluronic acid degraded by WT and the variant proteins.
FIG. 7E shows the results of analyzing WT and the variants HM40, HM13, HM41, HM24 and HM42 by phenyl column chromatography.
FIG. 7F shows the change in particle size with increasing temperature for the PH20 variants HM40, HM13, HM41 and HM42.
FIG. 8 shows experimental results for C-terminally truncated variants HM14, HM15 and HM16 constructed using HM6 as a template.
FIG. 8A shows the results of SDS-PAGE after HisTrap purification for the variants HM14, HM15 and HM16. As controls, WT and the variant HM6 were included.
FIG. 8B shows the results of measuring the enzymatic activities of WT and the variants HM6, HM14, HM15, and HM16 at pH 7.0 by turbidimetric assay.
FIG. 8C shows the results of measuring the enzymatic activities of WT and the variants HM6, HM14, HM15, and HM16 for 1, 2 and 4 h by substrate-gel assay. The right graph of FIG. 8C is a bar graph showing enzymatic activities measured after staining with Alcian-blue after 1 h of enzymatic reaction. The white band shows hyaluronic acid degraded by WT and the variant proteins.
FIG. 8D shows the results of analyzing WT and the variants HM6, HM14, HM15, and HM16 by phenyl column chromatography.
FIG. 8E shows the results of separating the variants HM14, HM15, and HM16 depending on their isoelectric points at the pH of 3 to 7 by IEF gel.
FIG. 9 shows experimental results for PH20 variants HM19 and HM20 constructed using HM10 as a template.
FIG. 9A shows the results of purified protein by HisTrap column chromatography for the PH20 variants HM19 and HM20.
FIG. 9B shows the results of comparing the enzymatic activities of the PH20 variant HM19 and HM20 at pH 7.0 by turbidimetric assay.
FIG. 9C shows the results of staining SDS gel with Alcian blue dye after 1 h of enzymatic reaction at 37°C by substrate-gel assay for WT and the variants HM10, HM19, and HM20. The white band shows hyaluronic acid degraded by WT and the variant proteins.
FIG. 10 shows the results of measuring the aggregation temperatures of WT and the PH20 variants by dynamic light scattering (hereinafter referred to as DLS) system. The measurements were performed in triplicate and expressed as mean ± S.E. values.

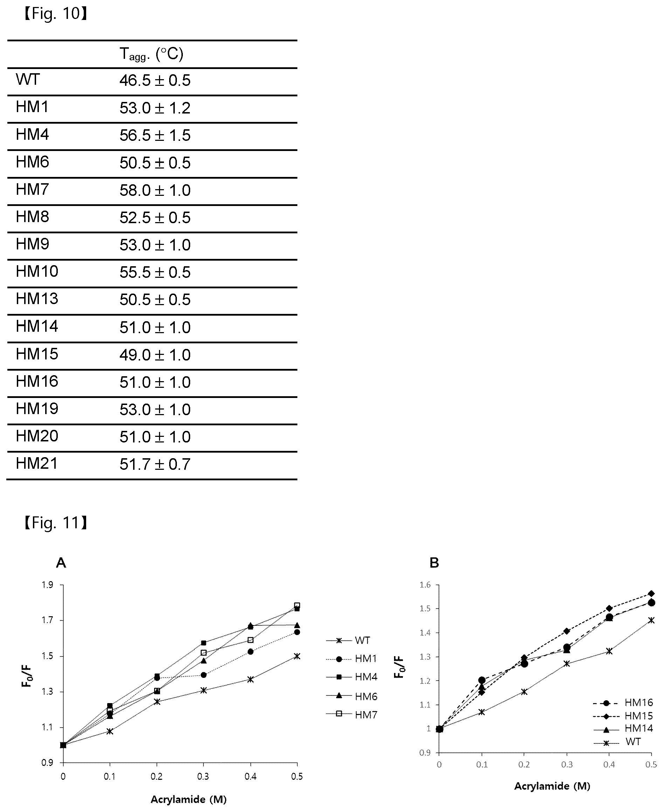

FIG. 11 shows a Stern-Volmer plot obtained after measuring the change in fluorescence of tryptophan residues of WT and PH20 variants by addition of acrylamide (0 to 0.5 M). Among amino acids, tryptophan is excited at 295 nm and emits maximum fluorescence wavelength at 340 nm. Acrylamide is a small molecule that can penetrate a protein structure and quench the fluorescence emission of tryptophan. As the protein structure is more flexible, the quenching of fluorescence by acrylamide is greater. F0 is the fluorescence value in the absence of acrylamide, and F is the fluorescence value in the presence of acrylamide (0 to 0.5 M). The change in the fluorescence value measured was expressed as the ratio F0/F.
FIG. 11A is a Stern-Volmer plot for WT and variants HM1, HM4, HM6 and HM7.
FIG. 11B is a Stern-Volmer plots for WT and variants HM14, HM15 and HM16.
FIG. 12 shows the expression levels of HM10-based PH20 variants in ExpiCHO cells.
FIG. 12A graphically shows the expression levels of respective variants.
FIG. 12B shows the expression levels of respective variants in the table. WT and the PH20 variants had a 6xHis-tag at the C-terminus, and the protein expression levels after HisTrap column purification were expressed in mg/L. HM30 to HM33 variants were not expressed in ExpiCHO cells.
FIG. 13 shows Western blot results for cell cultures of variants HM29, HM30, HM31, HM32 and HM33. The C-termini of HM10-based variants HM29, HM30, HM31, HM32 and HM33 were truncated after A467, C464, D461, C358, and C455, respectively. HM29 whose C-terminus was truncated at A467 was expressed in ExpiCHO cells, but variants having a C-terminus truncated at C464 or shorter in length were not expressed in ExpiCHO cells. Primary antibody was rabbit anti-PH20 polyclonal antibody (Abcam) diluted at 1:500. Secondary antibody was Goat anti-rabbit IgG HRP diluted at 1:2,000.
FIG. 14 shows experimental results for C-terminally truncated variants constructed using HM10 as a template.
FIG. 14A shows the results of measuring enzymatic activities at pH 7.0 by turbidimetric assay for C-terminally truncated variants constructed using HM10 as a template.
FIG. 14B compares enzymatic activities depending on the C-terminal truncation sites of 17 PH20 variants (HM43, HM44, HM45, HM20, HM19, HM35, HM36, HM37, HM38, HM39, HM47, HM48, HM49, HM50, HM51, HM52 and HM10) constructed using HM10 as a template.
FIG. 14C shows the results of staining SDS gel with Alcian blue dye after 1 h of enzymatic reaction at 37°C by substrate-gel assay for some (HM29, HM35, HM36, HM37, HM38, HM39, HM43, HM44 and HM45) of PH20 variants constructed using HM10 as a template. The white band shows hyaluronic acid degraded by WT and the variant proteins.
FIG. 15 shows SDS gel after passage through a final column during protein purification for the HP34 (FIG. 15A) and HP46 (FIG. 15B) expressed in ExpiCHO cells. HP34 was subjected to a four-step chromatography purification procedure consisting of Q Sepharose, Butyl HP, Heparin and Blue Sepharose columns, and SDS gel is a result obtained after Blue Sepharose column chromatography. HP46 was subjected to a three-step chromatography purification procedure consisting of Q Sepharose, Butyl HP and Heparin columns, and SDS gel is a result obtained after Heparin column chromatography.
FIG. 16 shows the enzymatic activities of 6xHis-tag-free PH20 variants HP34 and HP46 constructed using HM21 as a template.
FIG. 16A shows the results of measuring the enzymatic activities of WT and the variants HM21, HP34 and HP46 at pH 7.0 by turbidimetric assay.
FIG. 16B shows the results of measuring the enzymatic activities of HW2 and the variants HM21, HP34 and HP46 at pH 5.3 by Morgan-Elson assay (*K*ₘ: Michaelis-Menten constant, *k*_{cat}: turnover number, and *k*_{cat} / *K*ₘ: catalytic efficiency) .
FIG. 17 shows the results of characterization of HM21-Based PH20 Variants.
FIG. 17A shows the results of measuring aggregation temperatures by DLS for 6xHis-tag-free PH20 variants HP34 and HP46 constructed using HM21 as a template. As controls, the aggregation temperatures of HW2 and HM21 are shown.
FIG. 17B shows the results of measuring enzymatic activities for 1 h by substrate-gel assay for HW2 and the PH20 variants (HP20, HP34 and HP46).
FIG. 17C shows the results of performing a substrate-gel assay after allowing HW2 and HP46 samples to stand at pH 3.0 and pH 7.0 for 14 h. After SDS-PAGE, SDS was removed with 2.5% Triton X-100 (w/v), and an enzymatic reaction was performed at 37°C for 1 h.
FIG. 17D shows the expression levels of PH20 variants HM21, HM53, HM54, HM55, HM56, HP59 and HP60 in ExpiCHO cells.
FIG. 17E shows the expression of the enzymatic activity value of PH20 variants HM21, HM53, HM54, HM55, HM56, HP59 and HP60, measured by the turbidimetric assay, as specific activity at pH 5.3.
FIG. 18 shows the results of measuring the stimulating index of CD4+ T cells in treatment with PH20 and PH20 variant at concentrations of 1.5 ng/mL and 15 ng/mL, respectively.
FIG. 19 shows the results of measuring the stimulating index of CD8+ T cells in treatment with PH20 and PH20 variant at concentrations of 1.5 ng/mL and 15 ng/mL, respectively.

### Detailed Description and Preferred Embodiments of the Invention

Unless defined otherwise, all the technical and scientific terms used herein have the same meaning as those generally understood by one of ordinary skill in the art to which the invention pertains. Generally, the nomenclature used herein is well-known and commonly used in the art.

The present invention provides a hyaluronidase PH20 variant or fragment thereof, which comprises one or more amino acid residue substitutions in the region corresponding to an alpha-helix region and/or its linker region, preferably an alpha-helix 8 region (S347 to C381) and/or a linker region (A333 to R346) between alpha-helix 7 and alpha-helix 8, in the amino acid sequence of wild-type PH20, preferably mature wild-type PH20, and in which portion(s) of the N-terminal and/or C-terminal amino acid residues are selectively deleted by truncation.

In the present invention, the positions of amino acid residues in each variant correspond to the amino acid positions of wild-type PH20 having the sequence of SEQ ID NO: 1.

In addition, in the present invention, "mature wild-type PH20" means a protein consisting of amino acid residues L36 to S490 of SEQ ID NO: 1, which lacks M1 to T35, which form a signal peptide, and A491 to L509, which are not related to the substantial function of PH20, in the amino acid sequence of wild-type PH20 having the sequence of SEQ ID NO: 1.

**Table 1. Amino acid sequence of wild-type PH20 (SEQ ID NO: 1)**

| |
|---|
| MGVLKFKHIFFRSFVKSSGVSQIVFTFLLIPCCLTLNFRAPPVIPNVPFLWAWNAPSEFCLGKFDEP LDMSLFSFIGSPRINATGQGVTIFYVDRLGYYPYIDSITGVTVNGGIPQKISLQDHLDKAKKDITFY MPVDNLGMAVIDWEEWRPTWARNWKPKDVYKNRSIELVQQQNVQLSLTEATEKAKQEFEKAGKDFLV ETIKLGKLLRPNHLWGYYLFPDCYNHHYKKPGYNGSCFNVEIKRNDDLSWLWNESTALYPSIYLNTQ QSPVAATLYVRNRVREAIRVSKIPDAKSPLPVFAYTRIVFTDQVLKFLSQDELVYTFGETVALGASG IVIWGTLSIMRSMKSCLLLDNYMETILNPYIINVTLAAKMCSQVLCQEQGVCIRKNWNSSDYLHLNP DNFAIQLEKGGKFTVRGKPTLEDLEQFSEKFYCSCYSTLSCKEKADVKDTDAVDVCIADGVCIDAFL KPPMETEEPQIFYNASPSTLSATMFIVSILFLIISSVASL |

Specifically, the PH20 variant or fragment thereof according to the present invention may comprise one or more mutations, preferably amino acid residue substitutions selected from the group consisting of T341A, T341C, T341G, S343E, M345T, K349E, L353A, L354I, N356E and I361T, more preferably selected from the group consisting of T341A, T341C, L354I and N356E in wild-type PH20 having the amino acid sequence of SEQ ID NO: 1.

In the present invention, the term "PH20 variant" is intended to include mutation of portion(s) of amino acid residues, preferably substitution of one or more amino acid residues in the amino acid sequence of wild-type PH20, as well as occurrence of deletion of portion(s) of amino acid residues at N-terminus or C-terminus together with substitution of the amino acid residues, and is used as substantially the same meaning as the expression "PH20 variant or fragment thereof".

In the present invention, the protein tertiary structure of PH20 located outside the active site was studied through the protein structure modeling of human PH20 on the basis of Hyal1 (SEQ ID NO: 2) which is a human hyaluronidase whose protein tertiary structure is known. As a result, amino acids located in the alpha-helix 8 region of PH20 were selected and substituted with the amino acid sequence of alpha-helix 8 of Hyal1, thereby attempting to enhance the thermal stability of the protein structure without affecting the catalytic activity of the enzyme. In particular, alpha-helix 8 is located in the outer portion of the protein tertiary structure of PH20, and there is less interaction with the adjacent alpha-helix or beta-strand than the other alpha-helices of PH20. According to the present invention, it has been found that when the amino acid sequence of the alpha-helix 8 region of human PH20 and a linker region between alpha-helix 7 and alpha-helix 8 is partially substituted with the amino acid sequence of the alpha-helix 8 region of highly hydrophilic Hyal1 and a linker region between alpha-helix 7 and alpha-helix 8 of Hyal1, the enzymatic activity at neutral pH and the protein aggregation temperature (T_{agg}.) increase. Based on these experimental results, it has been found that a novel PH20 variant or fragment thereof, which has increased enzymatic activity and thermal stability compared to wild-type PH20, can be provided.

Thus, the PH20 variant according to the present invention comprises one or more amino acid residue substitutions selected from the group consisting of T341A, T341C, T341G, S343E, M345T, K349E, L353A, L354I, N356E and I361T, preferably selected from the group consisting of T341A, T341C, L354I and N356E in the amino acid sequence of wild-type PH20 (having the amino acid sequence of SEQ ID NO: 1), preferably mature wild-type PH20 (having a sequence consisting of amino acid residues L36 to S490 in the amino acid sequence of SEQ ID NO: 1).

The PH20 variant according to the present invention also comprises one or more amino acid residue substitutions in the region corresponding to an alpha-helix region and/or its linker region, preferably an alpha-helix 8 region (S347 to C381) and/or the linker region (A333 to R346) between alpha-helix 7 and alpha-helix 8, more preferably T341 to N363, T341 to I361, L342 to I361, S343 to I361, I344 to I361, M345 to I361, or M345 to N363.

In particular, in the PH20 variant according to the present invention, the alpha-helix 8 region (S347 to C381) and/or the linker region (A333 to R346) between alpha-helix 7 and alpha-helix 8 of the wild-type PH20, preferably the mature wild-type PH20 may be substituted with portion(s)of amino acid residues of the corresponding region (see Tables 2 and 3) of Hyal1 having the sequence of SEQ ID NO: 2, but is not limited thereto.

**Table 2. Amino acid sequence of wild-type Hyall (SEQ ID NO: 2)**

| |
|---|
| MAAHLLPICALFLTLLDMAQGFRGPLLPNRPFTTVWNANTQWCLERHGVDVDVSVFDVVANPGQTFR GPDMTIFYSSQLGTYPYYTPTGEPVFGGLPQNASLIAHLARTFQDILAAIPAPDFSGLAVIDWEAWR PRWAFNWDTKDIYRQRSRALVQAQHPDWPAPQVEAVAQDQFQGAARAWMAGTLQLGRALRPRGLWGF YGFPDCYNYDFLSPNYTGQCPSGIRAQNDQLGWLWGQSRALYPSIYMPAVLEGTGKSQMYVQHRVAE AFRVAVAAGDPNLPVLPYVQIFYDTTNHFLPLDELEHSLGESAAQGAAGVVLWVSWENTRTKESCQA IKEYMDTTLGPFILNVTSGALLCSQALCSGHGRCVRRTSHPKALLLLNPASFSIQLTPGGGPLSLRG ALSLEDQAQMAVEFKCRCYPGWQAPWCERKSMW |

**Table 3. Comparison of alpha-helices and amino acid sequence between PH20 and Hyall**

| alpha-helix | amino acid sequences of PH20 | amino acid sequences of Hyal1 |
|---|---|---|
| alpha-helix 1 | P56-D65 | N39-G48 |
| alpha-helix 3 | S119-M135 | S101-I117 |
| alpha-helix 4' | K161-N176 | K144-H159 |
| alpha-helix 4 | S180-R211 | P163-R194 |
| alpha-helix 5 | F239-S256 | P222-S239 |
| alpha-helix 6 | A274-D293 | K257-G277 |
| alpha-helix 7 | S317-G332 | P299-G314 |
| alpha-helix 8 | S347-C381 | T329-C363 |

More specifically, the novel PH20 variant or fragment thereof according to the present invention preferably comprises an amino acid residue substitution of L354I and/or N356E in the amino acid sequence of wild-type PH20, preferably mature wild-type PH20,

and further comprises at least the amino acid residue substitution at one or more positions selected from among T341 to N363, particularly one or more positions selected from the group consisting of T341, L342, S343, I344, M345, S347, M348, K349, L352, L353, D355, E359, I361 and N363, but is not limited thereto.

More preferably, the amino acid residue substitution at one or more positions selected from the group consisting of T341, L342, S343, I344, M345, S347, M348, K349, L352, L353, D355, E359, I361 and N363 may be one or more amino acid residue substitutions selected from the group consisting of T341A, T341C, T341D, T341G, T341S, L342W, S343E, I344N, M345T, S347T, M348K, K349E, L352Q, L353A, D355K, E359D, I361T and N363G , but is not limited thereto.

Preferably, the novel PH20 variant or fragment thereof according to the present invention may comprise amino acid residue substitutions of M345T, S347T, M348K, K349E, L352Q, L353A, L354I, D355K, N356E, E359D and I361T,

and may further comprise one or more amino acid residue substitutions selected from the group consisting of T341A, T341C, T341D, T341G, T341S, L342W, S343E, I344N and N363G, but is not limited thereto.

More preferably, the novel PH20 variant or fragment thereof according to the present invention may be any one selected from the following amino acid residue substitution groups, but is not limited thereto:
(a) T341S, L342W, S343E, I344N, M345T, S347T, M348K, K349E, L352Q,
   L353A, L354I, D355K, N356E, E359D and I361T;
(b) L342W, S343E, I344N, M345T, S347T, M348K, K349E, L352Q, L353A,
   L354I, D355K, N356E, E359D and I361T;
(c) M345T, S347T, M348K, K349E, L352Q, L353A, L354I, D355K, N356E, E359D, I361T and N363G;
(d) T341G, L342W, S343E, I344N, M345T, S347T, M348K, K349E, L352Q, L353A, L354I, D355K, N356E, E359D and I361T;
(e) T341A, L342W, S343E, I344N, M345T, S347T, M348K, K349E, L352Q, L353A, L354I, D355K, N356E, E359D and I361T;
(f) T341C, L342W, S343E, I344N, M345T, S347T, M348K, K349E, L352Q, L353A, L354I, D355K, N356E, E359D and I361T;
(g) T341D, L342W, S343E, I344N, M345T, S347T, M348K, K349E, L352Q, L353A, L354I, D355K, N356E, E359D and I361T;
(h) I344N, M345T, S347T, M348K, K349E, L352Q, L353A, L354I, D355K, N356E, E359D and I361T; and
(i) S343E, I344N, M345T, S347T, M348K, K349E, L352Q, L353A, L354I, D355K, N356E, E359D and I361T.

In the present invention, an expression described by one-letter amino acid residue code together with numbers, such as "S347", means the amino acid residue at each position in the amino acid sequence of SEQ ID NO: 1.

For example, "S347" means that the amino acid residue at position 347 in the amino acid sequence of SEQ ID NO: 1 is serine.

In addition, "S347T" means that serine at position 347 of SEQ ID NO: 1 is substituted with threonine.

The PH20 variant or fragment thereof according to the present invention is interpreted as including variants or fragments thereof in which the amino acid residue at a specific amino acid residue position is conservatively substituted.

As used herein, the term "conservative substitution" refers to modifications of a PH20 variant that involves the substitution of one or more amino acids for amino acids having similar biochemical properties that do not result in loss of the biological or biochemical function of the PH20 variant.

A "conservative amino acid substitution" is one in which the amino acid residue is replaced with an amino acid residue having a similar side chain. Families of amino acid residues having similar side chains have been defined and are well known in the art to which the present invention pertains. These families include amino acids with basic side chains (e.g., lysine, arginine and histidine), amino acids with acidic side chains (e.g., aspartic acid and glutamic acid), amino acids with uncharged polar side chains (e.g., glycine, asparagine, glutamine, serine, threonine, tyrosine, and cysteine), amino acids with nonpolar side chains (e.g., alanine, valine, leucine, isoleucine, proline, phenylalanine, methionine, and tryptophan), amino acids with beta-branched side chains (e.g., threonine, valine, and isoleucine), and amino acids with aromatic side chains (e.g., tyrosine, phenylalanine, tryptophan, and histidine).

It is envisioned that the PH20 variant or fragments thereof of the present invention may still retain the activity although it has conservative amino acid substitutions.

In addition, the PH20 variant or fragment thereof according to the present invention is interpreted to include PH20 variants or fragments thereof having substantially the same function and/or effect as those/that of the PH20 variant or fragment thereof according to the present invention, and having an amino acid sequence homology of at least 80% or 85%, preferably at least 90%, more preferably at least 95%, most preferably at least 99% to the PH20 variant or fragment thereof according to the present invention.

The PH20 variants or fragments thereof according to the present invention have increased expression levels in animal cells and an increased protein refolding rate, thereby increasing high thermal stability, compared to mature wild-type PH20. Furthermore, the enzymatic activity of the PH20 variants or fragments thereof was more increased than or similar to that of mature wild-type PH20 despite an increase in the thermal stability.

Meanwhile, it is known that when the C-terminal region of the mature wild type PH20 is truncated, the enzymatic activity is reduced. However, the PH20 variants according to the present invention, due to the increased protein refolding and thermal stability, exhibited similar or increased enzymatic activities compared to the mature wild type PH 20 despite the C-terminal being truncated. In addition, PH20 variants in this present invention maintained the enzymatic activities when the N-terminal amino acids were truncated up to five amino acids. This indicated that for the protein expression and enzyme activities, the role of amino acid residues from P41 of the N-terminus are important.

Accordingly, the PH20 variant or fragment thereof according to the present invention is characterized in that it comprises portion(s) of amino acid residue substitutions in the alpha-helix 8 region (S347 to C381) and/or the linker region (A333 to R346) between alpha-helix 7 and alpha-helix 8 in the amino acid sequence of wild-type PH20, and one or more of the N-terminal and/or C-terminal amino acid residues are additionally deleted, but is not limited thereto.

In one aspect, the PH20 variant or fragment thereof according to the present invention may be one in which truncation occurs before an amino acid residue selected from the group consisting of M1 to P42 at the N-terminus of the amino acid sequence of SEQ ID NO: 1, preferably before an amino acid residue L36, N37, F38, R39, A40, P41, or P42, so that portion(s) of amino acid residues at the N-terminus are deleted, and/or truncation occurs after an amino acid residue selected from the group consisting of V455 to L509, preferably after an amino acid residue selected from the group consisting of V455 to S490, most preferably after an amino acid residue V455, C458, D461, C464, I465, D466, A467, F468, K470, P471, P472, M473, E474, T475, E476, P478, I480, Y482, A484, P486, T488, or S490 at the C-terminus, so that portion(s) of amino acid residues at the C-terminus are deleted.

The expression "truncation occurs before an amino acid residue selected from the group consisting of M1 to P42 at the N-terminus" means that amino acid residues up to the amino acid residue which immediately precedes the amino acid residue selected from among M1 to P42 at the N-terminus are deleted by truncation.

For example, the expression "truncation occurs before an amino acid residue L36, N37, F38, R39, A40, P41, or P42" respectively means that all amino acid residues from M1 to T35 immediately before L36, all amino acid residues from M1 to L36 immediately before N37, all amino acid residues from M1 to N37 immediately before F38, all amino acid residues from M1 to F38 immediately before R39, all amino acid residues from M1 to R39 immediately before A40, all amino acid residues from M1 to A40 immediately before P41, or all amino acid residues from M1 to P41 immediately before P42 in the amino acid sequence of SEQ ID NO: 1 are removed by truncation, respectively.

In addition, the expression "truncation occurs after an amino acid residue selected from the group consisting of V455 to L509 at the C-terminus" means that amino acid residues starting with the amino acid residue which immediately follows the amino acid residue selected from among V455 to L509 at the C-terminus are deleted by truncation.

For example, the expression "truncation occurs after an amino acid residue V455, C458, D461, C464, I465, D466, A467, F468, K470, P471, P472, M473, E474, T475, E476, P478, I480, Y482, A484, P486, T488 or S490 at the C-terminus" means that amino acid residues starting with the amino acid residue which immediately follows the amino acid residue V455, C458, D461, C464, I465, D466, A467, F468, K470, P471, P472, M473, E474, T475, E476, P478, I480, Y482, A484, P486, T488 or S490, respectively, in the amino acid sequence of SEQ ID NO: 1 are removed by truncation.

Preferably, the novel PH20 variant or fragment thereof according to the present invention may be selected from the group consisting of amino acid sequences of SEQ ID NOs: 60 to 115, but is not limited thereto.

Most preferably, the novel PH20 variant or fragment thereof according to the present invention may have the amino acid sequence of SEQ ID NO: 99. The novel PH20 variant having an amino acid sequence of SEQ ID NO: 99 may comprise 15 amino acid residue substitutions of T341S, L342W, S343E, I344N, M345T, S347T, M348K, K349E, L352Q, L353A, L354I, D355K, N356E, E359D and I361T, and deletion before F38 residue at the N-terminus, and truncation after F468 residue at the C-terminus.

The sequences of the substituted or truncated amino acids in the PH20 variants constructed in the specific embodiments according to the present invention are as shown in Table 11.

A study focused on increasing the enzymatic activity and thermal stability of PH20 by amino acid substitution of an alpha-helix and its linker region, which are secondary structures forming the tertiary structure of the protein, as disclosed in the present invention, has not been previously reported. Previous studies reported that the enzymatic activity of wild-type PH20 changes depending on the truncation positions of amino acid residues located at the C-terminal region. However, in the present invention, a specific alpha-helix forming the secondary structure of PH20 was substituted with the alpha-helix of other human hyaluronidase, thereby constructing PH20 variants having higher stability than wild-type PH20. These variants may be variants in which the interaction of the substituted alpha-helix domain with portions forming other secondary structures of PH20 shows a pattern different from that of wild-type PH20, indicating that the variants have consistent enzymatic activity regardless of the C-terminal truncation position.

In specific embodiment, the novel PH20 variant or fragment thereof according to the present invention, which has increased enzymatic activity and thermal stability compared to mature wild-type PH20, may be one which comprises one or more amino acid residue substitutions selected from the group consisting of T341A, T341C, T341G, S343E, M345T, K349E, L353A, L354I, N356E and I361T, and in which portion(s) of amino acids located in an alpha-helix 8 region (S347 to C381) and/or the linker region (A333 to R346) between alpha-helix 7 and alpha-helix 8 in the amino acid sequence of wild-type PH20, preferably mature wild-type PH20, are substituted with other amino acids.

Specifically, the amino acid substitution in the alpha-helix 8 and the linker region between alpha-helix 7 and alpha-helix 8 comprises the substitution of portion(s) of amino acids in the region of amino acids T341 to N363, T341 to I361, L342 to I361, S343 to I361, I344 to I361, M345 to I361, or M345 to N363.

In order to examine the effect of C-terminal truncation in PH20 variants in which alpha-helix 8 and a linker region between alpha-helix 7 and alpha-helix 8 are substituted, three PH20 variants (HM6, HM10 and HM21) were selected as templates.

HM6 is a variant in which amino acids in M345 to N363 region are substituted with the amino acid sequence of Hyal1 (substitutions of M345T, S347T, M348K, K349E, L352Q, L353A, L354I, D355K, N356E, E359D and I361T in SEQ ID NO: 1). In addition, HM6 is a variant in which the substitution of alpha-helix 8 and a linker region between alpha-helix 7 and alpha-helix 8 is the least substituted variant among the PH20 variants according to the present invention, which do not comprise additional C-terminal truncation (that is, a form in which the C-terminal amino acid residue is S490, like mature wild-type PH20);

HM10 is a variant in which amino acids in L342 to I361 region are substituted with the amino acid sequence of Hyal1 (substitutions of L342W, S343E, I344N, M345T, S347T, M348K, K349E, L352Q, L353A, L354I, D355K, N356E, E359D and I361T in SEQ ID NO: 1), and which has the highest thermal stability while having an enzymatic activity similar to that of mature wild-type PH20 among the PH20 variants according to the present invention, which do not comprise additional C-terminal truncation; and

HM21 is a variant in which amino acids in T341 to I361 region are substituted with the amino acid sequence of Hyal1 (substitutions of T341S, L342W, S343E, I344N, M345T, S347T, M348K, K349E, L352Q, L353A, L354I, D355K, N356E, E359D and I361T in SEQ ID NO: 1), which does not comprise additional C-terminal truncation, and which has an enzymatic activity which is about two times higher than that of wild-type PH20 at pH 7.0.

The HM6-based PH20 variants constructed in the present invention have the N-terminus truncated at L36 and the C-terminus truncated after I465, F468, or P471 as shown in Table 4 below.

**Table 4. C-terminal amino acid truncated PH20 variants constructed using HM6 as a template**

| **Variant** | **Total length** | **Substituted region** | **Number of substituted amino acids** | **Number of truncated amino acids** |
|---|---|---|---|---|
| HM6 | 36-490 | | 11 | 0 |
| HM14 | 36-465 | M345-I361 | 11 | 25 |
| HM15 | 36-468 | | 11 | 22 |
| HM16 | 36-471 | | 11 | 19 |

The HM10-based PH20 variants commonly have the N-terminus truncated before the F38 residue and the C-terminus truncated after the V455, C4578, D46, C464, I465, D466, A467, F468, K470, P472, M473, E474, T475, E476, P478, I480, Y482, A484, P486, or T488 residue as shown in Table 5 below.

- [193]

**Table 5. C-terminal amino acid truncated PH20 variants constructed using HM10 as a template**

| **Variant** | **Total length** | **Substituted region** | **Number of substituted amino acids** | **Number of truncated amino acids** |
|---|---|---|---|---|
| HM10 | 36-490 | | 14 | 0 |
| HM52 | 38-488 | | 14 | 4 |
| HM51 | 38-486 | | 14 | 6 |
| HM50 | 38-484 | | 14 | 8 |
| HM49 | 38-482 | | 14 | 10 |
| HM48 | 38-480 | | 14 | 12 |
| HM47 | 38-478 | L342-I361 | 14 | 14 |
| HM39 | 38-476 | | 14 | 16 |
| HM38 | 38-475 | | 14 | 17 |
| HM37 | 38-474 | | 14 | 18 |
| HM36 | 38-473 | | 14 | 19 |
| HM35 | 38-472 | | 14 | 20 |
| HM19 | 38-470 | | 14 | 22 |
| HM20 | 38-468 | | 14 | 24 |
| HM45 | 38-467 | | 14 | 25 |
| HM29 | 36-467 | | 14 | 23 |
| HM44 | 38-466 | | 14 | 26 |
| HM43 | 38-465 | | 14 | 27 |
| HM30 | 36-464 | | 14 | 26 |
| HM31 | 36-461 | | 14 | 29 |
| HM32 | 36-458 | | 14 | 32 |
| HM33 | 36-455 | | 14 | 35 |
| HP19 | 38-470 | | 14 | 22 |
| HP20 | 38-468 | | 14 | 24 |

As shown in the examples of variants which comprise amino acid substitutions in the L342 to I361 region corresponding to the alpha-helix 8 region and the linker region between alpha-helix 7 and alpha-helix 8 of HM10 as a template and in which the N-terminus was truncated before the F38 residue and the C-terminus was truncated at I465, D466, A467, F468, K470, P472, M473, E474, T475, E476, P478, I480, Y482, A484, P486, or T488, the PH20 variants according to the present invention exhibited an enzymatic activity similar to that of mature wild-type PH20 regardless of the C-terminal truncation position.

As shown in Table 6 below, two HM21-based PH20 variants commonly have the N-terminus truncated before the F38 residue and the C-terminus truncated after the F468 or K470 residue.

**Table 6. C-terminal amino acid truncated PH20 variants constructed using HM21 as a template**

| **Variant** | **Total length** | **Substituted region** | **Number of substituted amino acids** | **Number of truncated amino acids** |
|---|---|---|---|---|
| HM21 | 38-490 | | 15 | 2 |
| HP34 | 38-470 | T341-I361 | 15 | 22 |
| HP46 | 38-468 | | 15 | 24 |

Variants were constructed using, as a template, HM21 having an enzymatic activity which is about two times higher than that of mature wild-type PH20. These variants are those which comprise amino acid substitutions in the T341 to I361 region corresponding to the alpha-helix 8 region and the linker region between alpha helix 7 and alpha-helix 8 and in which the N-terminus was truncated before the F38 residue, and the C-terminus was truncated after F468 or K470. Surprisingly, these variants maintained the high enzymatic activity of HM21 regardless of the C-terminal truncation position.

In the study conducted by Frost et al., when the length of PH20 is shorter due to the truncation before the amino acid position of 477, the enzymatic activity decreased to about 10% of a variant having a C-terminus truncated after the position 477(Frost, 2007). However, in the present invention, when amino acids in alpha-helix 8 of PH20 and its linker region were substituted, the enzymatic activity was maintained regardless of the C-terminal truncation position due to the increase in the stability of the protein. This result is very significant in that it solves the problem that the enzymatic activity of wild-type PH20 is reduced due to the C-terminal truncation of wild-type PH20.

In addition, in the present invention, the effect of the N-terminal amino acids of PH20, which has not been known previously, was studied.

In order to examine the effects of N-terminal truncation of amino acids in HM6 variants in which portion(s) of amino acid residues in the region (M345 to I361) corresponding to the alpha-helix 8 region of wild-type PH20 and the linker region between alpha-helix 7 and alpha-helix 8 were substituted with amino acid residues of the corresponding alpha-helix 8 region of Hyal1 and the linker region between alpha-helix 7 and alpha-helix 8 (i.e., substitutions of M345T, S347T, M348K, K349E, L352Q, L353A, L354I, D355K, N356E, E359D or I361T in SEQ ID NO: 1 and comprising no additional C-terminal truncation), variants, in which amino acid residues L36 to V47 in SEQ ID NO: 1 were substituted with FRGPLLPNR or amino acid residues L36 to A52 in SEQ ID NO: 1 were substituted with FRGPLLPNRPFTTV, were constructed using HM6 as a template. In addition, using HM6 as a template, the variants HM40, HM13, HM41, HM24, HM42 and HM25 were constructed in which the N-terminus in the amino acid sequence of SEQ ID NO: 1 was truncated before the N37, F38, R39, A40, P41 or P42 residue (see Table 7).

**Table 7. N-terminal truncated variants based on HM6**

| **Variant** | **Total length** | **Substituted region** | **Number of substituted amino acids** | **Number of truncated amino acids** |
|---|---|---|---|---|
| HM6 | 36-490 | M345-I361 | 11 | 0 |
| HM40 | 37-490 | | 11 | 1 |
| HM13 | 38-490 | | 11 | 2 |
| HM41 | 39-490 | | 11 | 3 |
| HM24 | 40-490 | | 11 | 4 |
| HM42 | 41-490 | | 11 | 5 |
| HM25 | 42-490 | | 11 | 6 |
| HM17 | | L36-V47, M345-I361 | 23 | |
| HM18 | | L36-A52, M345-I361 | 28 | |

As a result, it was shown that when the N-terminus of HM6 was truncated before the N37, F38, R39, A40 or P41 residue, the enzymatic activity was not greatly influenced; however, when the N-terminus was truncated before the P42 residue, the enzymatic activity significantly decreased, indicating that the N-terminal region of PH20, located after P41, is important for protein expression and enzymatic activity. In addition, when amino acids in the N-terminal L36 to V47 or L36 to A52 region of HM6 were substituted with the amino acids of Hyal1, the variant protein was not expressed in ExpiCHO cells, indicating that the N-terminal region is important for protein expression.

In addition, in the present invention, it was attempted to increase the expression of a recombinant PH20 protein by using the signal peptide of other proteins having a high level of protein expression in animal cells, instead of using the signal peptide specific to PH20.

Therefore, in another aspect, the novel PH20 variant according to the present invention may be one in which the N-terminus further comprises a human growth hormone signal peptide having an amino acid sequence MATGSRTSLLLAFGLLCLPWLQEGSA of SEQ ID NO: 3, a human serum albumin signal peptide having an amino acid sequence MKWVTFISLLFLFSSAYS of SEQ ID NO: 4, or a human Hyal1 signal peptide having an amino acid sequence MAAHLLPICALFLTLLDMAQG of SEQ ID NO: 5, as shown in Table 8 below, instead of the signal peptide of wild-type PH20, which consists of M1 to T35, but is not limited thereto.

The expression "instead of the signal peptide of wild-type PH20, which consists of M1 to T35" means a case in which the signal peptide of wild-type PH20 is partially or completely deleted; thus, it does not perform its function. In addition, the expression is meant to include a case in which portion(s) of the N-terminus is further deleted, for example, a case in which truncation occurs before the N37, F38, R39, A40, P41 or P42 residue occurs so that an additional deletion of the N-terminus together with the deletion of the signal peptide of wild-type PH20 occurs.

**Table 8. Amino acid sequences of signal peptide of human growth hormone, human serum albumin or human Hyal1**

| | **Amino acid sequences** | SEQ NO. |
|---|---|---|
| human growth hormone | MATGSRTSLLLAFGLLCLPWLQEGSA | 3 |
| human serum albumin | MKWVTFISLLFLFSSAYS | 4 |
| human Hyal1 | MAAHLLPICALFLTLLDMAQG | 5 |

In another aspect, the present invention provides a composition for treating cancer comprising the novel PH20 variant according to the present invention, and a method for treating cancer using the same.

Cancers or carcinomas that can be treated by the novel PH20 variant according to the present invention are not limited particularly, but includes both solid cancers and blood cancers. The cancer may be selected from the group consisting of skin cancer such as melanoma, liver cancer, hepatocellular carcinoma, gastric cancer, breast cancer, lung cancer, ovarian cancer, bronchial cancer, nasopharyngeal cancer, laryngeal cancer, pancreatic cancer, bladder cancer, colorectal cancer, colon cancer, cervical cancer, brain cancer, prostate cancer, bone cancer, thyroid cancer, parathyroid cancer, kidney cancer, esophageal cancer, biliary tract cancer, testicular cancer, rectal cancer, head and neck cancer, spinal cancer, ureteral cancer, osteosarcoma, neuroblastoma, fibrosarcoma, rhabdomyosarcoma, astrocytoma, neuroblastoma and glioma, but is not limited thereto. Preferably, cancers that can be treated by the composition according to the present invention may be selected from the group consisting of colorectal cancer, breast cancer, lung cancer, and kidney cancer, but are not limited thereto.

The composition of the present invention may be a pharmaceutical composition. The pharmaceutical composition may further comprise a pharmaceutically acceptable carrier. The carrier that is typically used in the formulation of drugs may be one or more selected from the group consisting of, but not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrups, methyl cellulose, methyl-hydroxybenzoate, propyl-hydroxybenzoate, talc, magnesium stearate, and mineral oil. In addition, the pharmaceutical composition may further comprise one or more selected from the group consisting of diluents, excipients, lubricants, wetting agents, sweeteners, aromatics, emulsifiers, suspensions, and preservatives which are commonly used in manufacturing pharmaceutical compositions.

The pharmaceutical composition of the present invention may be administered orally or parenterally. The parenteral administration is carried out by intravenous injection, subcutaneous injection, intramuscular injection, intraperitoneal injection, endothelial administration, topical administration, intranasal administration, intrapulmonary administration, rectal administration, and the like. For the oral administration, the active ingredient in the composition needs to be formulated into a coated dosage form or into a dosage form which can protect the active ingredient from being disintegrated in stomach considering that peptides and proteins are digested in stomach. In addition, the present composition may be administered via any device by which the active ingredient can move to the target cell of interest.

The pharmaceutical composition may be formulated in the form of solutions, suspensions, syrups or emulsions in oils or aqueous media, or in the form of extracts, grains, powders, granules, tablets or capsules, and may additionally include dispersing or stabilizing agents for the purpose of formulation.

In particular, the composition for treating cancer according to the present invention is characterized by its use in combined treatment with other anticancer drugs.

An anticancer drug that can be used for combined treatment with the novel PH20 variant according to the present invention is preferably a chemical anticancer drug, an antibody-based anticancer drug, an RNAi, or a cell therapeutic agent, but is not limited thereto.

The anticancer drug that can be used for combined treatment with the novel PH20 variant according to the present invention is preferably an immuno-oncologic agent, more preferably an immune checkpoint inhibitor, but is not limited thereto.

In another aspect, the present invention relates to a nucleic acid encoding the PH20 variant or a variant thereof.

The nucleic acids, as used herein, may be present in cells, in the cell lysate, or in the partially purified or substantially pure form. "Isolated" or "substantially pure", when referring to nucleic acids, refer to those that have been purified away from other cellular components or other contaminants, e.g., other cellular nucleic acids or proteins, by standard techniques, including alkaline/SDS treatment, CsCl banding, column chromatography, agarose gel electrophoresis, and others well known in the art. The nucleic acids of the present invention may be DNA or RNA.

In still another aspect, the present invention relates to vector comprising the nucleic acid. For expression of the PH20 variant or fragment thereof according to the present invention, a DNA encoding the PH20 variant can be obtained by standard molecular biology techniques (e.g., PCR amplification or cDNA cloning using a hybridoma that expresses the PH20 variant), and the DNA can be inserted into an expression vector such that it is "operably linked" to transcriptional and translational control sequences.

As used herein, the term "operably linked" is intended to mean that a gene encoding the PH20 variant or fragment thereof is ligated into a vector such that transcriptional and translational control sequences serve their intended function of regulating the transcription and translation of the gene encoding the PH20 variant or fragment thereof. The expression vector and expression control sequences are chosen to be compatible with the expression host cell used. The genes encoding the PH20 variant are inserted into the expression vector by standard methods (e.g., ligation of complementary restriction enzyme sites on a fragment of the gene encoding the PH20 variant or fragment thereof and vector, or blunt end ligation if no restriction enzyme sites are present).

In addition, the recombinant expression vectors carry regulatory sequences that control the expression of a gene encoding the PH20 variant in the host cell. The term "regulatory sequence" may include promoters, enhancers and other expression control elements (e.g., polyadenylation signals) that control the transcription or translation of the genes encoding the PH20 variant or fragment thereof. It will be appreciated by those skilled in the art that the design of the expression vector, including the selection of regulatory sequences, may depend on such factors as the choice of the host cell to be transformed, the level of expression of protein desired, etc.

In yet another aspect, the present invention relates to a host cell comprising the nucleic acid or the vector. The host cell according to the present invention is preferably selected from the group consisting of animal cells, plant cells, yeasts, *E*. *coli,* and insect cells, but is not limited thereto.

Specifically, the host cell according to the present invention may be prokaryotic cells such as *E*. *coli, Bacillus subtilis, Streptomyces* sp., *Pseudomonas* sp., *Proteus mirabilis* or *Staphylococcus* sp., fungi such as *Aspergillus* sp., yeasts such as *Pichia pastoris, Saccharomyces cerevisiae, Schizosaccharomyces* sp. and *Neurospora crassa,* and eukaryotic cells such as lower eukaryotic cells, and higher other eukaryotic cells such as insect cells.

In addition, the host cells may be derived from plants or mammals. Preferably, examples of the host cells include, but are not limited to, monkey kidney cells (COS7), NSO cells, SP2/0, Chinese hamster ovary (CHO) cells, W138, baby hamster kidney (BHK) cells, MDCK, myeloma cells, HuT 78 cells and HEK293 cells. More preferably, CHO cells may be used.

The nucleic acid or the vector is transfected into a host cell. "Transfection" can be performed using various techniques that are generally used to introduce foreign nucleic acid (DNA or RNA) into prokaryotic or eukaryotic cells, for example, electrophoresis, calcium phosphate precipitation, DEAE-dextran transfection or lipofection. In order to express the PH20 variant or fragment thereof of the present invention, various combinations of expression vectors and host cells can be employed. The preferred expression vector for eukaryotic cells comprises expression regulatory sequences derived from, but not limited to, SV40, bovine papillomavirus, adenovirus, adeno-associated virus, cytomegalovirus and retrovirus. The expression vector, which can be used for bacterial hosts, comprises bacterial plasmids, such as, pET, pRSET, pBluescript, pGEX2T, pUC vector, col E1, pCR1, pBR322, pMB9 and the derivatives thereof, obtained from *E*. *coli;* a plasmid having broad host range, such as, RP4; phage DNAs exemplified by various phage lambda derivatives, such as, λgt10, λgt11 and NM989; and other DNA phages, such as, M13 and filamentous single-stranded DNA phage. The expression vector available for yeast cells may be 2°C plasmid and its derivatives. The expression vector for insect cells is pVL941.

In a further aspect, the present invention relates to a method for producing a PH20 variant or fragment thereof, the method comprising a step of culturing the host cell and expressing the PH20 variant or fragment thereof according to the present invention.

When a recombinant expression vector capable of expressing the PH20 variant or fragment thereof is introduced into mammalian host cells, the PH20 variant or fragment thereof can be produced by culturing the host cells for a period of time such that the PH20 variant or fragment thereof is expressed in the host cells, preferably a sufficient period of time such that the PH20 variant is secreted into the medium during culture of the host cells.

In some cases, the expressed PH20 variant can be isolated from the host cells and purified to homogeneity. Isolation or purification of the PH20 variant can be performed by conventional isolation/purification methods (e.g., chromatography) that are used for proteins. The chromatography may be one or more combinations selected from affinity chromatography, ion exchange chromatography, and hydrophobic chromatography, but is not limited thereto. In addition to the chromatography, a combination of filtration, ultrafiltration, salting out, dialysis, and the like may be used.

Hereinafter, the present invention will be described in further detail with reference to working examples. It will be obvious to a person having ordinary skill in the art that these working examples are illustrative purposes only and are not to be construed to limit or change the scope of the present invention.

### WORKING EXAMPLES

Hereinafter, the present invention will be described in further detail with reference to working examples. It will be obvious to a person having ordinary skill in the art that these working examples are illustrative purposes only and are not to be construed to limit or change the scope of the present invention.

### Working Example 1. Construction of PH20 Variants

For construction of PH20 variants, the cDNA (clone ID: hMU002604) of wild-type PH20 was purchased from the Korean Human Gene Bank. Wild-type PH20 encodes amino acids from L36 to S490. The PH20 gene was amplified by polymerase chain reaction (hereinafter referred to as PCR) and inserted into the *XhoI* and *NotI* restriction enzyme sites of a pcDNA3.4-TOPO vector. For expression in ExpiCHO cells, the signal peptide of human growth hormone, human serum albumin or human Hyal1 was used as a signal peptide instead of the original signal peptide of PH20. For protein purification using a HisTrap column, the DNA sequence of a 6xHis-tag was located at the 3'-end of the PH20 cDNA. The amino acid substitution of PH20 variants was performed using PCR method, and the amino acid substitution was confirmed by DNA sequencing.

The list of primers used in cloning of the PH20 variants are summarized in Table 9 below, and the specific sequences of the primers are summarized in Table 10 below.

- [252]

**Table 9. List of primers used in cloning of PH20 variants**

| Clone | Primer | | |
|---|---|---|---|
| | 1 | 2 | 3 |
| cB4202 | ALB-SP-Xho | ALB-PH20-MR | SPAM1-6H-Not |
| cB4203-HM1 | ALB-SP-Xho | PH20_M345-364-F | SPAM1-6H-not |
| cB4203-HM2 | ALB-SP-Xho | PH20_Y365-L380-F | SPAM1-7H-not |
| cB4203-HM3 | ALB-SP-Xho | PH20_M345-L380-F | SPAM1-8H-not |
| cB4203-HM4 | ALB-SP-Xho | B4203-HM4-F | SPAM1-9H-not |
| cB4203-HM5 | ALB-SP-Xho | B4203-HM5-F | SPAM1-10H-not |
| cB4203-HM6 | ALB-SP-Xho | PH20-G363N | SPAM1-6H-not |
| cB4203-HM7 | ALB-SP-Xho | PH20-G363N | SPAM1-12H-not |
| cB4203-HM8 | ALB-SP-Xho | cB4203-HM8-M | SPAM1-13H-not |
| cB4203-HM9 | ALB-SP-Xho | cB4203-HM9-M | SPAM1-14H-not |
| cB4203-HM10 | ALB-SP-Xho | cB4203-HM10-M | SPAM1-6H-Not |
| cB4203-HM11 | ALB-SP-Xho | 4203-HM11 | SPAM1-16H-not |
| cB4203-HM12 | ALB-SP-Xho | 4203-HM12 | SPAM1-17H-not |
| cB4203-HM13 | ALB-SP-Xho | SASP-LN-del-R | SPAM1-18H-not |
| cB4203-HM14 | ALB-SP-Xho | - | I465-6H-not |
| cB4203-HM15 | ALB-SP-Xho | - | F468-6H-not |
| cB4203-HM16 | ALB-SP-Xho | - | P471-6H-not |
| cB4203-HM17 | ALB-SP-Xho | PH20-HM17 | SPAM1-22H-not |
| cB4203-HM18 | ALB-SP-Xho | PH20-HM18 | SPAM1-23H-not |
| cB4203-HM19 | ALB-SP-Xho | SASP-LN-del-R | K470-6H-not |
| cB4003-HP19 | ALB-SP-Xho | K470-not | |
| cB4203-HM20 | ALB-SP-Xho | SASP-LN-del-R | F468-6H-not |
| cB4003-HP20 | ALB-SP-Xho | F468-not | |
| cB4203-HM21 | ALB-SP-Xho | M21-mega-F | SPAM1-6H-Not |
| cB4203-HM24 | ALB-SP-Xho | M24-R | SPAM1-6H-not |
| cB4203-HM25 | ALB-SP-Xho | M25-R | SPAM1-6H-not |
| cB4203-HM26 | ALB-SP-Xho | B4-HM26 | SPAM1-6H-not |
| cB4203-HM27 | ALB-SP-Xho | B4-HM27 | SPAM1-6H-not |
| cB4203-HM28 | ALB-SP-Xho | B4-HM28 | SPAM1-6H-not |
| cB4203-HM29 | ALB-SP-Xho | B4-HM29 | |
| cB4203-HM30 | ALB-SP-Xho | B4-HM30 | |
| cB4203-HM31 | ALB-SP-Xho | B4-HM31 | |
| cB4203-HM32 | ALB-SP-Xho | B4-HM32 | |
| cB4203-HM33 | ALB-SP-Xho | B4-m33 | |
| cB4003-HP34 | ALB-SP-Xho | M21-mega-F | K470-not |
| cB4203-HM35 | ALB-SP-Xho | SASP-LN-del-R | P472-6H-not |
| cB4003-HP35 | ALB-SP-Xho | P472-not | |
| cB4203-HM36 | ALB-SP-Xho | SASP-LN-del-R | M473-6H-not |
| cB4003-HP36 | ALB-SP-Xho | M473-not | |
| cB4203-HM37 | ALB-SP-Xho | SASP-LN-del-R | E474-6H-not |
| cB4003-HP37 | ALB-SP-Xho | E474 -not | |
| cB4203-HM38 | ALB-SP-Xho | SASP-LN-del-R | T475-6H-not |
| cB4003-HP38 | ALB-SP-Xho | T475-not | |
| cB4203-HM39 | ALB-SP-Xho | SASP-LN-del-R | E476-6H-not |
| cB4003-HP39 | ALB-SP-Xho | SASP-LN-del-R | E476-not |
| cB4203-HM40 | ALB-SP-Xho | M40-mega | SPAM1-6H-Not |
| cB4203-HM41 | ALB-SP-Xho | M41-mega | SPAM1-6H-Not |
| cB4203-HM42 | ALB-SP-Xho | m42-mega | SPAM1-6H-not |
| cB4203-HM43 | ALB-SP-Xho | I465-6H-not | |
| cB4203-HM44 | ALB-SP-Xho | D466-6H-not | |
| cB4203-HM45 | ALB-SP-Xho | B4-HM29 | |
| cB4003-HP46 | ALB-SP-Xho | F468-Not | |
| cB4203-HM47 | ALB-SP-Xho | P478-H-Not | |
| cB4203-HM48 | ALB-SP-Xho | I480-H-Not | |
| cB4203-HM49 | ALB-SP-Xho | Y482-H-Not | |
| cB4203-HM50 | ALB-SP-Xho | A484 -H-not | |
| cB4203-HM51 | ALB-SP-Xho | P486-H-not | |
| cB4203-HM52 | ALB-SP-Xho | SASP-LN-del-R | T488-H-not |

**Table 10. Primer sequences used in cloning of PH20 variants**

| Primer | SEQ NO. | Nucleotide Sequences (5'-> 3') |
|---|---|---|
| ALB-SP-Xho | 6 | GAA TAT CTC GAG GCC ACC ATG AAG TGG GTT ACA |
| SPAM1-6H-Not | 7 | CTA ATT GCG GCC GCT CAT TAG TGG TGA TGG TGA TGA TGG AAG AAA CCA ATT CTG C |
| ALB-PH20-MR | 8 | TAA CAG GAG GTG CTC TGA AAT TCA GAG AGT AAG CAG AGG AG |
| PH20_M345-P364-F | 9 | ATG GGG AAC CCT CAG TAT AAC AAG AAC CAA GGA ATC ATG TCA GGC CAT CAA GGA GTA TAT GGA CAC TAC ACT GGG GCC CTA CAT AAT CAA CGT CAC AC |
| PH20_Y365-L380-F | 10 | ATG GAG ACT ATA CTG AAT CCT TTC ATC CTG AAC GTG ACC AGT GGG GCC CTT CTC TGC AGT CAA GCC CTG TGC CAG GAG CAA GGA GTG TG |
| PH20_M345-L380-F* | 11 | ATG GAC ACT ACA CTG GGG CCC TTC ATC CTG AAC GTG ACC AGT GGG GCC CTT CTC TGC AGT CAA GCC CTG TGC CAG GAG CAA GGA GTG TG |
| B4203-HM4-F | 12 | ACT GTT GCT CTG GGT GCT TCT GGA ATT GTA ATA TGG GTA AGC TGG GAA AAT ACA AGA ACC AAG GAA TCA TGT CA |
| B4203-HM5-F | 13 | AGC AAG GAG TGT GTA TAA GGA AAA CCA GCC ACC CAA AAG ACT ATC TTC ACC TCA ACC CAG A |
| PH20-G363N | 14 | AGT ATA TGG ACA CTA CAC TGA ACC CCT ACA TAA TCA ACG TCA C |
| cB4203-HM8-M | 15 | ATT GTA ATA TGG GGA ACC CTC AGT AAT ACA AGA ACC AAG GAA TC |
| cB4203-HM9-M | 16 | ATT GTA ATA TGG GGA ACC CTC GAA AAT ACA AGA ACC AAG GAA TC |
| cB4203- HM10-M | 17 | ATT GTA ATA TGG GGA ACC TGG GAA AAT ACA AGA ACC AAG GAA TC |
| 4203-HM11 | 18 | ACA CTA CAC TGA ACC CCT TCA TAC TCA ACG TCA CCC TAG CAG CCA |
| 4203-HM12 | 19 | ACA CTA CAC TGA ACC CCT TCA TAC TCA ACG TCA CCC TAT CAG GCA AAA TGT GTA GCC AAG TGC |
| SASP-LN-del-R | 20 | TAA CAG GAG GTG CTC TGA AAG AGT AAG CAG AGG AG |
| I465-6H-not | 21 | CTA ATT GCG GCC GCT CAT TAG TGG TGA TGG TGA TGA TGT ATA CAG ACA CCA TCA GC |
| F468-6H-not | 22 | CTA ATT GCG GCC GCT CAT TAG TGG TGA TGG TGA TGA TGA AAA GCA TCT ATA CAG ACA CC |
| P471-6H-not | 23 | CTA ATT GCG GCC GCT CAT TAG TGG TGA TGG TGA TGA TGA GGT TTT AGA AAA GCA TCT ATA C |
| PH20-HM17 | 24 | TCC AGG CCC AGA GGA AAG GCC GGT TGG GTA GCA AGG GGC CCC TAA AAG AGT AAG CAG AGG AG |
| PH20-HM18 | 25 | TCA CTT GGG GCA TTC CAG ACG GTG GTG AAG GGC CGG TTG GGT AGC AAG GGG CCC CTA AAA GAG TAA GCA GAG GAG |
| K470-not | 26 | CTA ATT GCG GCC GCT CAT TAG TGG TGA TGG TGA TGA TGT TTT AGA AAA GCA TCT ATA C |
| F468-not | 27 | CTA ATT GCG GCC GCT CAT TAA AAA GCA TCT ATA CAG ACA CC |
| M21-mega-F | 28 | AAT TGT AAT ATG GGG AAG CTG GGA AAA TAC AAG AA |
| M24-R | 29 | TGG AAT AAC AGG AGG TGC AGA GTA AGC AGA GGA GA |
| M25-R | 30 | TTT GGA ATA ACA GGA GAG TAA GCA GAG GAG A |
| B4-HM26 | 31 | AGT TTT GAA ATT CCT TTC TCT GGA TGA GCT GGA GCA CAG CCT GGG GGA GAG TGC GGC CCA GGG TGC TTC TGG AAT TG |
| B4-HM27 | 32 | ATG AGC TGG AGC ACA GCT TTG GGG AGA GTG CGG CCC AG |
| B4-HM28 | 33 | ATT CCT TTC TCA AGA TGA ACT TGA GCA CAG CTT TGG CGA AAC TGT TGC |
| B4-HM29 | 34 | CTA ATT GCG GCC GCT CAT TAG TGG TGA TGG TGA TGA TGA GCA TCT ATA CAG ACA CC |
| B4-HM30 | 35 | CTA ATT GCG GCC GCT CAT TAG TGG TGA TGG TGA TGA TGA CAG ACA CCA TCA GCA ATA C |
| B4-HM31 | 36 | CTA ATT GCG GCC GCT CAT TAG TGG TGA TGG TGA TGA TGA TCA GCA ATA CAC ACA TC |
| B4-HM32 | 37 | CTA ATT GCG GCC GCT CAT TAG TGG TGA TGG TGA TGA TGA CAC ACA TCA ACA GCA TC |
| B4- HM33 | 38 | CTA ATT GCG GCC GCT CAT TAG TGG TGA TGG TGA TGA TGA ACA GCA TCA GTG TCT TTT AC |
| P472-not | 39 | GTT ATA GCG GCC GCT CAT TAG GGA GGT TTT AGA AAA GCA TC |
| M473-not | 40 | GTT ATA GCG GCC GCT CAT TAC ATG GGA GGT TTT AGA AAA GCA TC |
| E474-not | 41 | GTT ATA GCG GCC GCT CAT TAC TCC ATG GGA GGT TTT AGA AAA GC |
| T475-not | 42 | GTT ATA GCG GCC GCT CAT TAT GTC TCC ATG GGA GGT TTT AG |
| M40-mega | 43 | TAA CAG GAG GTG CTC TGA AAT TAG AGT AAG CAG AGG AG |
| M41-mega | 44 | TGG AAT AAC AGG AGG TGC TCT AGA GTA AGC AGA GGA G |
| M42-mega | 45 | TTT GGA ATA ACA GGA GGA GAG TAA GCA GAG GAG |
| D466-6H-not | 46 | CTA ATT GCG GCC GCT CAT TAG TGG TGA TGG TGA TGA TGA TCT ATA CAG ACA CCA TCA GC |
| P478-H-Not | 47 | GAT AAT GCG GCC GCT CAT TAG TGG TGA TGG TGA TGA TGA GGT TCT TCT GTC TCC ATG GG |
| I480-H-Not | 48 | GAT AAT GCG GCC GCT CAT TAG TGG TGA TGG TGA TGA TGA ATT TGA GGT TCT TCT GTC TCC |
| Y482-H-Not | 49 | GAT AAT GCG GCC GCT CAT TAG TGG TGA TGG TGA TGA TGG TAG AAA ATT TGA GGT TCT TCT G |
| A484 -H-not | 50 | GAT AAT GCG GCC GCT CAT TAG TGG TGA TGG TGA TGA TGA GCA TTG TAG AAA ATT TGA GGT TC |
| P486-H-not | 51 | GAT AAT GCG GCC GCT CAT TAG TGG TGA TGG TGA TGA TGG GGT GAA GCA TTG TAG AAA ATT TGA GG |
| T488-H-not | 52 | GAT AAT GCG GCC GCT CAT TAG TGG TGA TGG TGA TGA TGT GTG GAG GGT GAA GCA TTG TAG |
| K470-6H-not | 53 | CTA ATT GCG GCC GCT CAT TAG TGG TGA TGG TGA TGA TGT TTT AGA AAA GCA TCT ATA C |
| P472-6H-not | 54 | GTT ATA GCG GCC GCT CAT TAG TGG TGA TGG TGA TGA TGG GGA GGT TTT AGA AAA GCA TC |
| M473-6H-not | 55 | GTT ATA GCG GCC GCT CAT TAG TGG TGA TGG TGA TGA TGC ATG GGA GGT TTT AGA AAA GCA TC |
| E474-6H-not | 56 | GTT ATA GCG GCC GCT CAT TAG TGG TGA TGG TGA TGA TGC TCC ATG GGA GGT TTT AGA AAA GC |
| T475-6H-not | 57 | GTT ATA GCG GCC GCT CAT TAG TGG TGA TGG TGA TGA TGT GTC TCC ATG GGA GGT TTT AG |
| E476-6H-not | 58 | GTT ATA GCG GCC GCT CAT TAG TGG TGA TGG TGA TGA TGT TCT GTC TCC ATG GGA GG |
| E476-not | 59 | GTT ATA GCG GCC GCT CAT TAT TCT GTC TCC ATG GGA GG |

After finding the PH20 variant with increased enzymatic activity and thermal stability, the 6xHis-tag-free cDNA of the PH20 variant was also constructed.

When the cell density of ExpiCHO cells reached 6×10⁶/mL, a plasmid comprising the wild-type or variant PH20 cDNA inserted in the pcDNA3.4-TOPO vector was transfected into the ExpiCHO cells by ExpiFectamine CHO reagent. As a cell culture medium, ExpiCHO expression medium (100 to 500 mL) was used. After transfection, the ExpiCHO cells were shake-cultured at 130 rpm for a total of 6 days, during which the cells were cultured at 37°C for 1 day and further cultured at lower temperature of 32°C for 5 days. After completion of the culture, the cell supernatant was collected by centrifugation at 10,000 rpm for 30 min.

The recombinant proteins of the C-terminal 6xHis-attached wild-type PH20 and variant PH20, produced in the ExpiCHO cells, were purified in three steps (performed using a HisTrap column, a Q Sepharose column and a Phenyl column, respectively) by an AKTA prime system.

For protein purification using the HisTrap column, buffer A (20 mM sodium phosphate, pH 7.5, 0.5 M NaCl) and buffer B (20 mM sodium phosphate, pH 7.5, 0.5 M NaCl, 0.5 M imidazole) were prepared. The protein was bound to the HisTrap column, and the column was flushed with 5 column volumes (CV) of buffer A to remove non-specifically bound proteins. It was confirmed that the conductivity was maintained at constant level, the column was flushed with 5 CV of 20% buffer B to elute the protein. The eluted protein was dialyzed with dialysis buffer (20 mM sodium phosphate, pH 7.5, 50 mM NaCl). For protein purification using the Q Sepharose column, buffer A (20 mM sodium phosphate, pH 7.5) and buffer B (20 mM sodium phosphate, pH 7.5, 0.5 M NaCl) were prepared. The protein was bound to the Q Sepharose column, and the column was flushed with 5 CV of buffer A to remove nonspecifically bound proteins, and then 5 CV of buffer B was flushed at concentration gradient of 0 to 100% to elute the protein.

For protein purification using the phenyl column, buffer A (20 mM sodium phosphate, pH 7.0, 1.5 M (NH₄)₂SO₄) and buffer B (20 mM sodium phosphate, pH 7.0) were prepared. The protein was bound to the phenyl column, and the column was flushed with 5 CV of buffer A to remove nonspecifically bound proteins, and then 5 CV of buffer B was flushed at concentration gradient of 0 to 100% to elute the protein.

The enzymatic activities of wild-type PH20 and variant PH20 were measured by turbidimetric assay, substrate-gel assay, and Morgan-Elson assay.

The turbidimetric assay is a method of measuring the absorbance in the precipitate produced when hyaluronic acid is mixed with albumin (BSA). When hyaluronic acid is hydrolyzed by PH20, the absorbance of the precipitate produced when mixed with albumin decreases. Hyaluronidase PH20 (Sigma) was diluted to 1, 2, 5, 7.5, 10, 15, 20, 30, 50 and 60 units/mL, and prepared in each tube. The purified protein sample was dissolved in enzyme diluent buffer (20 mM Tris·HCl, pH 7.0, 77 mM NaCl, 0.01% (w/v) bovine serum albumin) and diluted to 100X, 300X, 600X, 1200X and 2400X and prepared in each tube. In fresh tubes, the hyaluronic acid solution having a concentration of 3 mg/mL was diluted 10-fold to a concentration of 0.3 mg/mL so that the volume of each tube became 180 µL. 60 µL of enzyme was added to and mixed with the diluted hyaluronic acid solution and allowed to react at 37°C for 45 min. After completion of the reaction, 50 µL of the reacted enzyme and 250 µL of acidic albumin solution were added to each well of a 96-well plate and shaken for 10 min, and then the absorbance was measured at 600 nm by spectrophotometer.

In the substrate-gel assay, the protein was electrophoresed on 10% SDS gel (including 0.17 mg/mL hyaluronic acid) for 1 h, and SDS was removed with 2.5% Triton X-100 (w/v) at 4°C for 2 h. Thereafter, an enzymatic reaction was performed in the buffer (50 mM sodium phosphate, pH 7.0, 150 mM NaCl) at 37°C (which is the optimum temperature for PH20) for 1 to 4 h, and the protein was stained with 0.5% Alcian blue reagent. Alcian blue reagent not bound to the hyaluronic acid was removed using de-staining solution. The SDS gel stained with Alcian blue was imaged, and then the band was quantified.

The thermal stability of the protein was measured by a method of measuring the aggregation temperature by dynamic light scattering (DLS), a method of measuring the melting temperature (Tₘ) in real-time PCR using Sypro-Orange dye, a method of measuring the enzymatic activity after leaving the protein to stand at predetermined temperature for a predetermined time, etc. In the method of measuring the aggregation temperature by DLS, the aggregation of molecules is measured using light scattering, and thus the sensitivity is high, and the aggregation temperature is generally lower than the melting temperature of the protein (Tₘ).

The sequences of the substituted or truncated amino acids in the PH20 variants constructed in the present invention are shown in Table 11 below.

Among the variants according to the present invention, the variant having the 6xHis-tag attached to the C-terminus of PH20 was named HM; the variant free of the 6xHis-tag was named HP; mature wild-type PH20 (L36 to S490) having the 6xHis-tag attached to the C-terminus was named WT; and mature wild-type PH20 (L36 to Y482) having the C-terminus truncated after Y482 while being free of the 6xHis-tag was named HW2.

-[303]

**Table 11. Amino acid sequences of PH20 variants according to the present invention and substitution/truncation characteristics thereof**

| Name | SEQ NO. | Substitution | Amino Acid Sequence |
|---|---|---|---|
| HM1 | 60 | 12 amino acid substitutions of M345T, S347T, M348K, K349E, L352Q, L353A, L354I, D355K, N356E, E359D, I361T and N363G. | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLSITRTKES CQAIKEYMDTTLGPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM2 | 61 | 7 amino acid substitutions of Y365F, I367L, L371S, A372G, K374L, M375L and V379A | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLSITRTKES CQAIKEYMDTTLNPFILNVTSGALLCSQALCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM3 | 62 | 19 amino acid substitutions of M345T, S347T, M348K, K349E, L352Q, L353A, L354I, D355K, N356E, E359D, I361T, N363G, Y365F, I367L, L371S, A372G, K374L, M375L and V379A | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLSITRTKES CQAIKEYMDTTLGPFILNVTSGALLCSQALCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM4 | 63 | 17 amino acid substitutions of G340V, T341S, L342W, S343E, I344N, M345T, S347T, M348K, K349E, L352Q, L353A, L354I, D355K, N356E, E359D, I361T and N363G | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWVSWENTRTKES CQAIKEYMDTTLGPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM6 | 64 | 11 amino acid residue substitutions of M345T, S347T, M348K, K349E, L352Q, L353A, L354I, D355K, N356E, E359D and I361T | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLSITRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM7 | 65 | 16 amino acid substitutions of G340V, T341S L342W, S343E, I344N, M345T, S347T, M348K, K349E, L352Q, L353A, L354I, D355K, N356E, E359D and I361T | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWVSWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM8 | 66 | 12 amino acid substitutions of I344N, M345T, S347T, M348K, K349E, L352Q, L353A, L354I, D355K, N356E, E359D and I361T | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLSNTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM9 | 67 | 13 amino acid substitutions of S343E, I344N, M345T, S347T, M348K, K349E, L352Q, L353A, L354I, D355K, N356E, E359D and I361T | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM10 | 68 | 14 amino acid residue substitutions of L342W, S343E, I344N, M345T, S347T, M348K, K349E, L352Q, L353A, L354I, D355K, N356E, E359D and I361T | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM11 | 69 | 13 amino acid residue substitutions of M345T, S347T, M348K, K349E, L352Q, L353A, L354I, D355K, N356E, E359D, I361T, Y365F and I367L | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLSITRTKES CQAIKEYMDTTLNPFILNVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM12 | 70 | 15 amino acid residue substitutions of M345T, S347T, M348K, K349E, L352Q, L353A, L354I, D355K, N356E, E359D, I361T, Y365F, I367L, L371S and A372G | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLSITRTKES CQAIKEYMDTTLNPFILNVTSGAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM13 | 71 | 11 amino acid residue substitutions of M345T, S347T, M348K, K349E, L352Q, L353A, L354I, D355K, N356E, E359D and I361T, and truncation before F38 residue at the N-terminus | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTLSITRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPMETEEPQIFYNASPSTLS |
| HM14 | 72 | 11 amino acid substitutions of M345T, S347T, M348K, K349E, L352Q, L353A, L354I, D355K, N356E, E359D and I361T and truncation after I465 at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLSITRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCI |
| HM15 | 73 | 11 amino acid substitutions of M345T, S347T, M348K, K349E, L352Q, L353A, L354I, D355K, N356E, E359D and I361T, and truncation after F468 at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLSITRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAF |
| HM16 | 74 | 11 amino acid substitutions of M345T, S347T, M348K, K349E, L352Q, L353A, L354I, D355K, N356E, E359D and I361T, and truncation after P471 at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTLSITRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKP |
| HM17 | 75 | Substitution of L36-V47 with FRGPLLPNR, and 11 amino acid substitutions of M345T, S347T, M348K, K349E, L352Q, L353A, L354I, D355K, N356E, E359D and I361T | FRGPLLPNRPFLWAWNAPSEFCLGKFDEPLDMSLF SFIGSPRINATGQGVTIFYVDRLGYYPYIDSITGV TVNGGIPQKISLQDHLDKAKKDITFYMPVDNLGMA VIDWEEWRPTWARNWKPKDVYKNRSIELVQQQNVQ LSLTEATEKAKQEFEKAGKDFLVETIKLGKLLRPN HLWGYYLFPDCYNHHYKKPGYNGSCFNVEIKRNDD LSWLWNESTALYPSIYLNTQQSPVAATLYVRNRVR EAIRVSKIPDAKSPLPVFAYTRIVFTDQVLKFLSQ DELVYTFGETVALGASGIVIWGTLSITRTKESCQA IKEYMDTTLNPYIINVTLAAKMCSQVLCQEQGVCI RKNWNSSDYLHLNPDNFAIQLEKGGKFTVRGKPTL EDLEQFSEKFYCSCYSTLSCKEKADVKDTDAVDVC IADGVCIDAFLKPPMETEEPQIFYNASPSTLS |
| HM18 | 76 | Substitution of L36-A52 with FRGPLLPNRPFTTV, and 11 amino acid substitutions of M345T, S347T, M348K, K349E, L352Q, L353A, L354I, D355K, N356E, E359D and I361T | FRGPLLPNRPFTTVWNAPSEFCLGKFDEPLDMSLF SFIGSPRINATGQGVTIFYVDRLGYYPYIDSITGV TVNGGIPQKISLQDHLDKAKKDITFYMPVDNLGMA VIDWEEWRPTWARNWKPKDVYKNRSIELVQQQNVQ LSLTEATEKAKQEFEKAGKDFLVETIKLGKLLRPN HLWGYYLFPDCYNHHYKKPGYNGSCFNVEIKRNDD LSWLWNESTALYPSIYLNTQQSPVAATLYVRNRVR EAIRVSKIPDAKSPLPVFAYTRIVFTDQVLKFLSQ DELVYTFGETVALGASGIVIWGTLSITRTKESCQA IKEYMDTTLNPYIINVTLAAKMCSQVLCQEQGVCI RKNWNSSDYLHLNPDNFAIQLEKGGKFTVRGKPTL EDLEQFSEKFYCSCYSTLSCKEKADVKDTDAVDVC IADGVCIDAFLKPPMETEEPQIFYNASPSTLS |
| HM19 | 77 | 14 amino acid residue substitutions of L342W, S343E, I344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLK |
| | | truncation before F38 residue at the N-terminus, and truncation after K470 residue at the C-terminus | |
| HM20 | 78 | 14 amino acid residue substitutions of L342W, S343E, I344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAF |
| | | truncation before F38 residue at the N-terminus, and truncation after F468 residue at the C-terminus | |
| HM21 | 79 | 15 amino acid substitutions of T341S, L342W, S343E, I344N, M345T, S347T, M348K, K349E, L352Q, L353A, L354I, D355K, N356E, E359D and I361T | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGSWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM24 | 80 | 11 amino acid residue substitutions of M345T, S347T, M348K, K349E, L352Q, L353A, L354I, D355K, N356E, E359D and I361T, and truncation before A40 residue at the N-terminus | APPVIPNVPFLWAWNAPSEFCLGKFDEPLDMSLFS FIGSPRINATGQGVTIFYVDRLGYYPYIDSITGVT VNGGIPQKISLQDHLDKAKKDITFYMPVDNLGMAV IDWEEWRPTWARNWKPKDVYKNRSIELVQQQNVQL SLTEATEKAKQEFEKAGKDFLVETIKLGKLLRPNH LWGYYLFPDCYNHHYKKPGYNGSCFNVEIKRNDDL SWLWNESTALYPSIYLNTQQSPVAATLYVRNRVRE AIRVSKIPDAKSPLPVFAYTRIVFTDQVLKFLSQD ELVYTFGETVALGASGIVIWGTLSITRTKESCQAI KEYMDTTLNPYIINVTLAAKMCSQVLCQEQGVCIR KNWNSSDYLHLNPDNFAIQLEKGGKFTVRGKPTLE DLEQFSEKFYCSCYSTLSCKEKADVKDTDAVDVCI ADGVCIDAFLKPPMETEEPQIFYNASPSTLS |
| HM25 | 81 | 11 amino acid substitutions of M345T, S347T, M348K, K349E, L352Q, L353A, L354I, D355K, N356E, E359D and I361T, truncation before P42 at the N-terminus | PVIPNVPFLWAWNAPSEFCLGKFDEPLDMSLFSFI GSPRINATGQGVTIFYVDRLGYYPYIDSITGVTVN GGIPQKISLQDHLDKAKKDITFYMPVDNLGMAVID WEEWRPTWARNWKPKDVYKNRSIELVQQQNVQLSL TEATEKAKQEFEKAGKDFLVETIKLGKLLRPNHLW GYYLFPDCYNHHYKKPGYNGSCFNVEIKRNDDLSW LWNESTALYPSIYLNTQQSPVAATLYVRNRVREAI RVSKIPDAKSPLPVFAYTRIVFTDQVLKFLSQDEL VYTFGETVALGASGIVIWGTLSITRTKESCQAIKE YMDTTLNPYIINVTLAAKMCSQVLCQEQGVCIRKN WNSSDYLHLNPDNFAIQLEKGGKFTVRGKPTLEDL EQFSEKFYCSCYSTLSCKEKADVKDTDAVDVCIAD GVCIDAFLKPPMETEEPQIFYNASPSTLS |
| HM29 | 82 | 14 amino acid residue substitutions of L342W, S343E, 1344N, M345T, S347T, M348K, K349E, L352Q, L353A, L354I, D355K, N356E, E359D and I361T, truncation before L36 residue at the N-terminus, and truncation after A467 residue at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDA |
| HM30 | 83 | 14 amino acid residue substitutions of L342W, S343E, 1344N, M345T, S347T, M348K, K349E, L352Q, L353A, L354I, D355K, N356E, E359D and I361T, truncation before L36 residue at the N-terminus, and truncation after C464 residue at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVC |
| HM31 | 84 | 14 amino acid residue substitutions of L342W, S343E, 1344N, M345T, S347T, M348K, K349E, L352Q, L353A, L354I, D355K, N356E, E359D and I361T, truncation before L36 residue at the N-terminus, and truncation after D461 residue at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIAD |
| HM32 | 85 | 14 amino acid residue substitutions of L342W, S343E, 1344N, M345T, S347T, M348K, K349E, L352Q, L353A, L354I, D355K, N356E, E359D and I361T, truncation before L36 residue at the N-terminus, and truncation after C458 residue at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVC |
| HM33 | 86 | 14 amino acid residue substitutions of L342W, S343E, 1344N, M345T, S347T, M348K, K349E, L352Q, L353A, L354I, D355K, N356E, E359D and I361T, truncation before L36 residue at the N-terminus, and truncation after V455 residue at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGTWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV |
| HP34 | 87 | 15 amino acid residue substitutions of T341S, L342W, S343E, I344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGSWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLK |
| | | truncation before F38 residue at the N-terminus, and truncation after K470 residue at the C-terminus | |
| HM35 | 88 | 14 amino acid residue substitutions of L342W, S343E, 1344N, M345T, S347T, M348K, K349E, L352Q, L353A, L354I, D355K, N356E, E359D and I361T, truncation before F38 residue at the N-terminus, and truncation after P472 residue at the C-terminus | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPP |
| HM36 | 89 | 14 amino acid residue substitutions of L342W, S343E, 1344N, M345T, S347T, M348K, K349E, L352Q, L353A, L354I, D355K, N356E, E359D and I361T, truncation before F38 residue at the N-terminus, and truncation after M473 residue at the C-terminus | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPM |
| HM37 | 90 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPME |
| | | truncation before F38 residue at the N-terminus, and truncation after E474 residue at the C-terminus | |
| HM38 | 91 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPMET |
| | | truncation before F38 residue at the N-terminus, and truncation after T475 residue at the C-terminus | |
| HM39 | 92 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPMETE |
| | | truncation before F38 residue at the N-terminus, and truncation after E476 residue at the C-terminus | |
| HM40 | 93 | 11 amino acid residue substitutions of M345T, S347T, M348K, K349E, L352Q, L353A, L354I, D355K, N356E, E359D and I361T, and | NFRAPPVIPNVPFLWAWNAPSEFCLGKFDEPLDMS LFSFIGSPRINATGQGVTIFYVDRLGYYPYIDSIT GVTVNGGIPQKISLQDHLDKAKKDITFYMPVDNLG MAVIDWEEWRPTWARNWKPKDVYKNRSIELVQQQN VQLSLTEATEKAKQEFEKAGKDFLVETIKLGKLLR PNHLWGYYLFPDCYNHHYKKPGYNGSCFNVEIKRN DDLSWLWNESTALYPSIYLNTQQSPVAATLYVRNR VREAIRVSKIPDAKSPLPVFAYTRIVFTDQVLKFL SQDELVYTFGETVALGASGIVIWGTLSITRTKESC QAIKEYMDTTLNPYIINVTLAAKMCSQVLCQEQGV CIRKNWNSSDYLHLNPDNFAIQLEKGGKFTVRGKP TLEDLEQFSEKFYCSCYSTLSCKEKADVKDTDAVD VCIADGVCIDAFLKPPMETEEPQIFYNASPSTLS |
| | | truncation before N37 residue at the N-terminus | |
| HM41 | 94 | 11 amino acid residue substitutions of M345T, S347T, M348K, K349E, L352Q, L353A, L354I, D355K, N356E, E359D and I361T, and | RAPPVIPNVPFLWAWNAPSEFCLGKFDEPLDMSLF SFIGSPRINATGQGVTIFYVDRLGYYPYIDSITGV TVNGGIPQKISLQDHLDKAKKDITFYMPVDNLGMA VIDWEEWRPTWARNWKPKDVYKNRSIELVQQQNVQ LSLTEATEKAKQEFEKAGKDFLVETIKLGKLLRPN HLWGYYLFPDCYNHHYKKPGYNGSCFNVEIKRNDD LSWLWNESTALYPSIYLNTQQSPVAATLYVRNRVR EAIRVSKIPDAKSPLPVFAYTRIVFTDQVLKFLSQ DELVYTFGETVALGASGIVIWGTLSITRTKESCQA IKEYMDTTLNPYIINVTLAAKMCSQVLCQEQGVCI RKNWNSSDYLHLNPDNFAIQLEKGGKFTVRGKPTL EDLEQFSEKFYCSCYSTLSCKEKADVKDTDAVDVC IADGVCIDAFLKPPMETEEPQIFYNASPSTLS |
| | | truncation before R39 residue at the N-terminus | |
| HM42 | 95 | 11 amino acid residue substitutions of M345T, S347T, M348K, K349E, L352Q, L353A, L354I, D355K, N356E, E359D and I361T, and | PPVIPNVPFLWAWNAPSEFCLGKFDEPLDMSLFSF IGSPRINATGQGVTIFYVDRLGYYPYIDSITGVTV NGGIPQKISLQDHLDKAKKDITFYMPVDNLGMAVI DWEEWRPTWARNWKPKDVYKNRSIELVQQQNVQLS LTEATEKAKQEFEKAGKDFLVETIKLGKLLRPNHL WGYYLFPDCYNHHYKKPGYNGSCFNVEIKRNDDLS WLWNESTALYPSIYLNTQQSPVAATLYVRNRVREA IRVSKIPDAKSPLPVFAYTRIVFTDQVLKFLSQDE LVYTFGETVALGASGIVIWGTLSITRTKESCQAIK EYMDTTLNPYIINVTLAAKMCSQVLCQEQGVCIRK NWNSSDYLHLNPDNFAIQLEKGGKFTVRGKPTLED LEQFSEKFYCSCYSTLSCKEKADVKDTDAVDVCIA DGVCIDAFLKPPMETEEPQIFYNASPSTLS |
| | | truncation before P41 residue at the N-terminus | |
| HM43 | 96 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGSWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCI |
| | | truncation before F38 residue at the N-terminus, and truncation after I465 residue at the C-terminus | |
| HM44 | 97 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGSWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCID |
| | | truncation before F38 residue at the N-terminus, and truncation after D466 residue at the C-terminus | |
| HM45 | 98 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGSWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDA |
| | | truncation before F38 residue at the N-terminus, and truncation after A467 residue at the C-terminus | |
| HP46 | 99 | 15 amino acid residue substitutions of T341S, L342W, S343E, I344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGSWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAF |
| | | truncation before F38 residue at the N-terminus, and truncation after F468 residue at the C-terminus | |
| HM47 | 100 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPMETEEP |
| | | truncation before F38 residue at the N-terminus, and truncation after P478 residue at the C-terminus | |
| HM48 | 101 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPMETEEPQI |
| | | truncation before F38 residue at the N-terminus, and truncation after 1480 residue at the C-terminus | |
| HM49 | 102 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPMETEEPQIFY |
| | | truncation before F38 residue at the N-terminus, and truncation after Y482 residue at the C-terminus | |
| HM50 | 103 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPMETEEPQIFYNA |
| | | truncation before F38 residue at the N-terminus, and truncation after A484 residue at the C-terminus | |
| HM51 | 104 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPMETEEPQIFYNASP |
| | | truncation before F38 residue at the N-terminus, and truncation after P486 residue at the C-terminus | |
| HM52 | 105 | 14 amino acid residue substitutions of L342W, S343E, 1344N, M345T, S347T, M348K, K349E, L352Q, L353A, L354I, D355K, N356E, E359D and I361T, | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAFLKPPMETEEPQIFYNASPST |
| | | truncation before F38 residue at the N-terminus, and truncation after T488 residue at the C-terminus | |
| HM53 | 106 | 15 amino acid residue substitutions of T341G, L342W, S343E, I344N, M345T, S347T, M348K, K349E, L352Q, L353A, L354I, D355K, N356E, E359D and I361T, truncation before L36 residue at the N-terminus, and truncation after S490 residue at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGGWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM54 | 107 | 15 amino acid residue substitutions of T341A, L342W, S343E, I344N, M345T, S347T, M348K, K349E, L352Q, L353A, L354I, D355K, N356E, E359D and I361T, truncation before L36 residue at the N-terminus, and truncation after S490 residue at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGAWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HM55 | 108 | 15 amino acid residue substitutions of T341C, L342W, S343E, I344N, M345T, S347T, M348K, K349E, L352Q, L353A, L354I, D355K, N356E, E359D and I361T, truncation before L36 residue at the N-terminus, and truncation after S490 residue at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGCWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQ109IFYNASPS TLS |
| HM56 | 109 | 15 amino acid residue substitutions of T341D, L342W, S343E, I344N, M345T, S347T, M348K, K349E, L352Q, L353A, L354I, D355K, N356E, E359D and I361T, truncation before L36 residue at the N-terminus, and truncation after S490 residue at the C-terminus | LNFRAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPVDNL GMAVIDWEEWRPTWARNWKPKDVYKNRSIELVQQQ NVQLSLTEATEKAKQEFEKAGKDFLVETIKLGKLL RPNHLWGYYLFPDCYNHHYKKPGYNGSCFNVEIKR NDDLSWLWNESTALYPSIYLNTQQSPVAATLYVRN RVREAIRVSKIPDAKSPLPVFAYTRIVFTDQVLKF LSQDELVYTFGETVALGASGIVIWGDWENTRTKES CQAIKEYMDTTLNPYIINVTLAAKMCSQVLCQEQG VCIRKNWNSSDYLHLNPDNFAIQLEKGGKFTVRGK PTLEDLEQFSEKFYCSCYSTLSCKEKADVKDTDAV DVCIADGVCIDAFLKPPMETEEPQIFYNASPSTLS |
| HP57 | 110 | 12 amino acid residue substitutions of 1344N, M345T, S347T, M348K, K349E, L352Q, L353A, L354I, D355K, N356E, E359D and I361T, truncation before F38 residue at the N-terminus, and truncation after F468 residue at the C-terminus | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTLSNTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAF |
| HP58 | 111 | 13 amino acid residue substitutions of S343E, I344N, M345T, S347T, M348K, K349E, L352Q, L353A, L354I, D355K, N356E, E359D and I361T, truncation before F38 residue at the N-terminus, and truncation after F468 residue at the C-terminus | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGTLENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAF |
| HP59 | 112 | 15 amino acid residue substitutions of T341A, L342W, S343E, I344N, M345T, S347T, M348K, K349E, L352Q, L353A, L354I, D355K, N356E, E359D and I361T, truncation before F38 residue at the N-terminus, and truncation after F468 resiue at the C-terminus | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGAWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAF |
| HP60 | 113 | 15 amino acid residue substitutions of T341G, L342W, S343E, I344N, M345T, S347T, M348K, K349E, L352Q, L353A, L354I, D355K, N356E, E359D and I361T, truncation before F38 residue at the N-terminus, and truncation after F468 residue at the C-terminus | FRAPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGGWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAF |
| HP61 | 114 | 16 amino acid residue substitutions of A40G, T341S, L342W, S343E, 1344N, M345T, S347T, M348K, K349E, L352Q, L353A, L354I, D355K, N356E, E359D and I361T, truncation before F38 residue at the N-terminus, and truncation after F468 residue at the C-terminus | FRGPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGSWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAF |
| HP62 | 115 | Removal of P42, 15 amino acid residue substitutions of T341S, L342W, S343E, I344N, M345T, S347T, M348K, K349E, L352Q, L353A, L354I, D355K, N356E, E359D and I361T, truncation before F38 residue at the N-terminus, and truncation after F468 residue at the C-terminus | FRGPPVIPNVPFLWAWNAPSEFCLGKFDEPLDMSL FSFIGSPRINATGQGVTIFYVDRLGYYPYIDSITG VTVNGGIPQKISLQDHLDKAKKDITFYMPVDNLGM AVIDWEEWRPTWARNWKPKDVYKNRSIELVQQQNV QLSLTEATEKAKQEFEKAGKDFLVETIKLGKLLRP NHLWGYYLFPDCYNHHYKKPGYNGSCFNVEIKRND DLSWLWNESTALYPSIYLNTQQSPVAATLYVRNRV REAIRVSKIPDAKSPLPVFAYTRIVFTDQVLKFLS QDELVYTFGETVALGASGIVIWGSWENTRTKESCQ AIKEYMDTTLNPYIINVTLAAKMCSQVLCQEQGVC IRKNWNSSDYLHLNPDNFAIQLEKGGKFTVRGKPT LEDLEQFSEKFYCSCYSTLSCKEKADVKDTDAVDV CIADGVCIDAF |

### Working Example 2. Construction of PH20 Variants HM1 and HM6

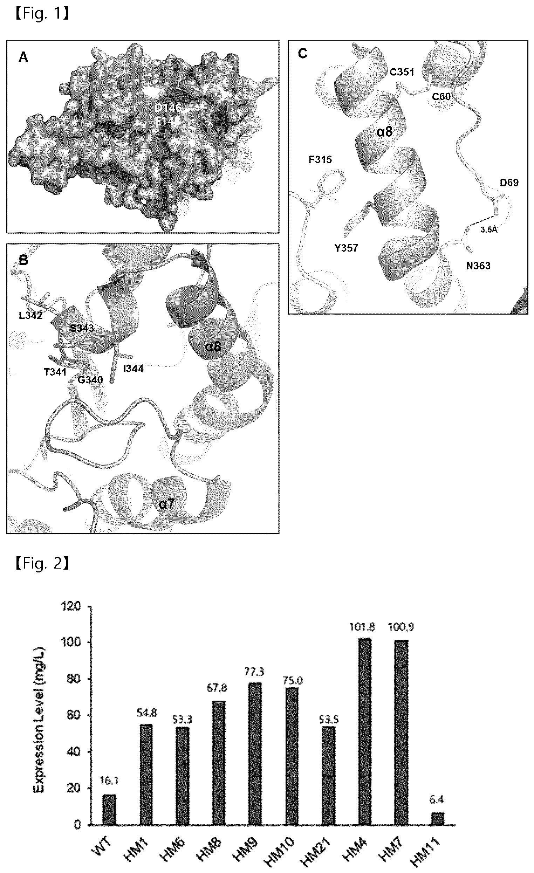

As shown in FIG. 1B, amino acid residues M345 to N363 of PH20 correspond to the alpha-helix 8 region and the linker region between alpha-helix 7 and alpha-helix 8 in the protein tertiary structure model. Among the amino acids of alpha-helix 8, C351 forms a disulfide bond with C60 of alpha-helix 1; Y357 forms hydrophobic interaction with F315 of alpha-helix 7; and N363 forms a hydrogen bond with D69 of alpha-helix 1, thereby stabilizing secondary structures adjacent to alpha-helix 8 (FIG. 1C).

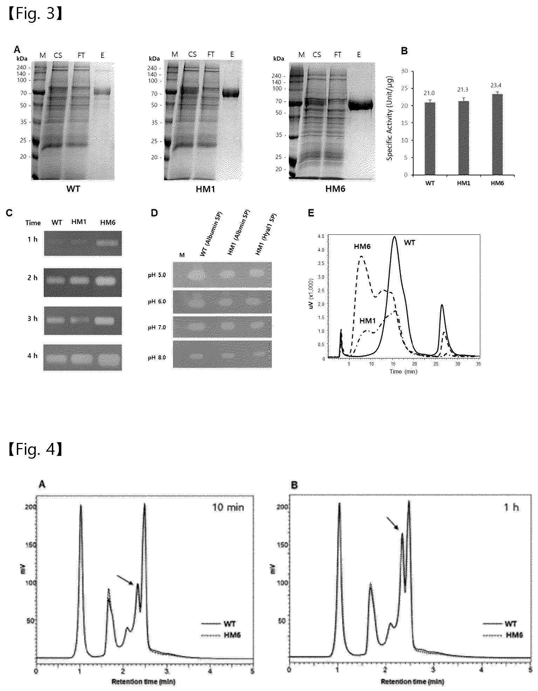

In order to construct variants having higher enzymatic activity and thermal stability than WT by substituting amino acids located in the alpha-helix 8 region of PH20 and the linker region between alpha-helix 7 and alpha-helix 8, the following variants were constructed: the variant HM1 in which 12 amino acids in the M345 to N363 region were substituted; the variant HM2 in which 7 amino acids in the Y365 to V379 region were substituted; and the variant HM3 in which 19 amino acids in the M345 to V379 region were substituted. Among the amino acids located in alpha-helix 8 of PH20, C351, which is involved in disulfide bonding, and Y357 which is involved in hydrophobic interaction, were not substituted. The substituted amino acid sequences in the variants HM1, HM2 and HM3 are shown in Table 11 above. When ExpiCHO cells were transfected with the pcDNA3.4-TOPO plasmid comprising the gene of the variant HM1, HM2 or HM3, the variant HM1 was expressed in the ExpiCHO cells (FIG. 3A), and the variants HM2 and HM3 were not expressed in the cells. Whether the protein was be expressed was confirmed not only by measurement of the enzymatic activity, but also by Western blot analysis using an antibody (Abcam, ab193009) against human PH20. The epitope of the antibody is Q173 to P222 region. These experimental results suggest that amino acid substitution of Y365 to V379 region of the amino acid sequence of alpha-helix 8, used in construction of the variants HM2 and HM3, causes a serious effect on the protein structure; thus, the proteins of the variants HM2 and HM3 were not expressed. The expression level of the variant HM1 was 3.4-fold higher than that of wild-type WT (FIG. 2). The variant HM1 is one in which the hydrogen bond between N363 of alpha-helix 8 and D69 of alpha-helix 1 was removed by substituting N363 with glycine (FIG. 1C). To restore the hydrogen bond between N363 and D69, G363 in HM1 was substituted with asparagine, thereby constructing the variant HM6. The substituted amino acids in the variant HM6 are shown in Table 11 above.

The variant HM6 was expressed in ExpiCHO cells, and the expression level thereof was similar to that of the variant HM1 (the expression level was 3.4-fold higher than that of WT) (FIG. 2). When the enzymatic activity was measured by turbidimetric assay, it was shown that the enzymatic activity of the variant HM6 was 1.3-fold higher than that of WT (FIG. 3B). In the substrate-gel assay, generally, SDS is removed with 2.5% Triton X-100 (w/v) after SDS-PAGE, and enzymatic reaction is performed at 37°C for 1 to 4 h, during which the extent to which hyaluronidase hydrolyzes hyaluronic acid is measured using Alcian blue dye. It is known that when SDS is removed from the substrate gel, protein refolding immediately occurs, and the substrate does not affect the protein refolding. When the enzymatic activities of WT and the variants HM1 and HM6 were measured by the substrate-gel assay at 37°C for 1 to 4 h, HM6 exhibited higher enzymatic activity than those of WT and the variant HM1 (FIG. 3C). This result suggests that protein refolding of the variants HM1 and HM6 and the resulting renaturation were faster than those of WT and that the constructed variants exhibit higher enzymatic activity than WT. When the signal peptide of PH20 itself was used, the expression level of the protein in ExpiCHO cells was low, and to solve this problem, the signal peptide sequence of human serum albumin or human Hyal1 was used. As shown in FIG. 3C, when each of the signal peptides of human serum albumin and human Hyal1 was used as the signal peptide of the variant HM1, the expression of the protein increased, and there was no significant difference between the two signal peptides. When the enzymatic activity was measured by the substrate-gel assay, WT having the signal peptide of human serum albumin and the variant HM1 having the signal peptide of human serum albumin or Hyal1 exhibited enzymatic activity at the pH ranging from 5 to 8 (FIG. 3D). In the present invention, the signal peptide of human serum albumin was used as the signal peptide of the variant constructed after HM1.

When the thermal stabilities of WT and the variants HM1 and HM6 were compared based on the aggregation temperatures, the aggregation temperatures were 46.5°C, 53.0°C and 50.5°C, respectively, indicating that the aggregation temperatures of the variants HM1 and HM6 were 6.5°C and 4.0°C higher, than that of WT, respectively (FIG. 10). The results of measuring the aggregation temperatures were consistent with the protein refolding results shown in the substrate-gel assay. These results suggest for the first time that the hydrogen bond formed by the N363 residue of alpha-helix 8 plays an important role in the thermal stability and enzymatic activity of PH20.

In addition, when the hydrophilic/hydrophobic natures of WT and the variants HM1 and HM6 were compared using a phenyl column chromatography, HM1 and HM6 were all eluted earlier than WT. This suggests that the variants HM1 and HM6 have a more hydrophilic nature than WT due to the substitution of the amino acids. However, the variants HM1 and HM6 eluted from the phenyl column showed two peaks, unlike WT, and exhibited the same molecular weight when treated with PNGase F. This appears to be a difference caused by N-glycosylation (Fig. 3E).

### Working Example 3. Construction of PH20 Variants HM4, HM7, HM8, HM9, HM10, HM11 and HM12

It is believed that the fact that the amino acid substitution of the M345 to N363 and M345 to I361 regions by construction of the variants HM1 and HM6 in Working Example 2 resulted in an increase in both the enzymatic activity and thermal stability of PH20 has made great advances in terms of protein engineering. Thus, based on the variants HM1 and HM6, other amino acids in the N-terminal and C-terminal directions were additionally substituted.

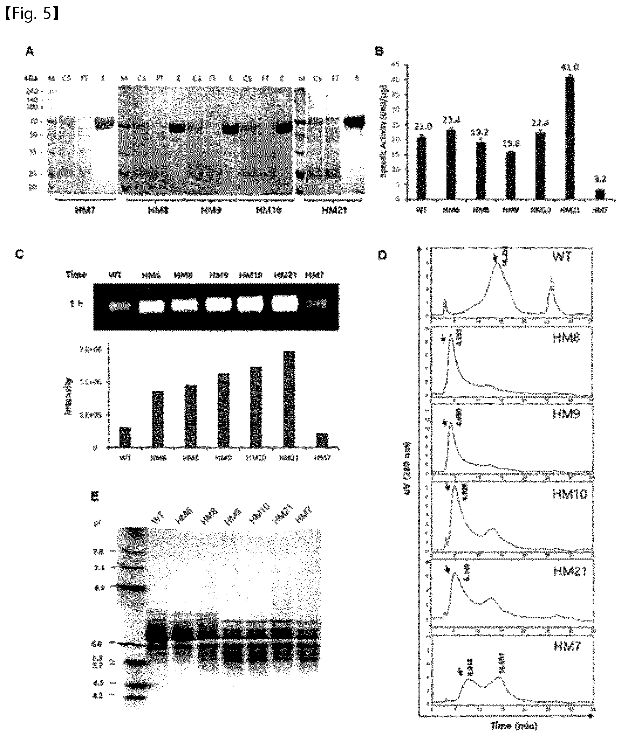

First, the variants HM4 and HM7 were constructed, which comprise the substituted amino acids in the variants HM1 and HM6 and in which the amino acids between G340 and I344 were additionally substituted with G340V, T341S, L342W, S343E and I344N. The sequences of the substituted amino acid sequences in the variants HM4 and HM7 are shown in Table 11 above. The variants HM4 and HM7 were expressed in ExpiCHO cells. The results of protein purification for HM7 are shown in FIG. 5A. HM4 and HM7 showed an increase in protein expression of 6.3-fold compared to WT, and showed increases in aggregation temperature of 10°C and 11.5°C, respectively, compared to WT. However, the enzymatic activities of HM4 and HM7, measured by turbidimetric assay, were merely about 15% of that of WT (FIG. 5B). Enzymatic activity and thermal stability generally have a trade-off relationship, but in the present invention, it appeared that the substitution introduced in the variants HM1 and HM6 increased the thermal stability of the variants while maintaining the enzymatic activity, and the enzymatic activities of the variants HM4 and HM7 decreased due to an excessive increase in the thermal stability. An increase in aggregation temperature of 11.5°C, which appeared in the variants HM4 and HM7 is a very significant result in terms of protein engineering. When the structural flexibilities of the proteins were analyzed by a Stern-Volmer plot, it was shown that the structural flexibilities of HM1, HM6, HM4 and HM7, obtained by substituting alpha-helix 8 and its linker region, were all higher than that of WT (FIG. 11A). This result suggests that the increase in local thermal stability resulted in an increase in the flexibility of the entire protein structure.

The difference between the variants HM6 and HM7 is amino acids between G340 and I344. In order to identify amino acids which are involved in the increased thermal stability of the variant HM7, the following variants were made based on the variant HM6: HM8 having substitution I344N; HM9 having substitutions S343 and 1344N; HM10 having substitutions L342W, S343E and I344N; and HM21 having substitutions T341S, L342W, S343E and I344N. The sequences of the substituted amino acids in the variants HM8, HM9, HM10 and HM21 are shown in Table 11 above. The variants HM8, HM9, HM10 and HM21 were expressed in ExpiCHO cells (FIG. 5A). As I344N, S343E and L342W were introduced in the N-terminal direction of alpha-helix 8 based on the variant HM6, the aggregation temperatures of the variants HM8, HM9 and HM10 increased to 52.5°C, 53°C and 55.5°C, respectively (FIG. 10). However, the variants HM8, HM9 and HM10 maintained enzymatic activities similar to that of WT (FIG. 5B). This result suggests that the amino acid substitutions introduced in the variants HM8, HM9 and HM10 had a local effect on the thermal stabilities of the enzymes but had no significant effect on the enzymatic activities. However, HM21 showed reduced thermal stability compared to HM10, but showed approximately 2-fold higher property compared to WT at pH 7.0. When WT and each variant were allowed to react with the substrate for 1 h in the substrate-gel assay, the enzymatic activity shown was in the order of HM21> HM10> HM9> HM8> HM6> WT (FIG. 5C).

When the physical properties of the variants HM7, HM8, HM9, HM10 and HM21 were examined using a phenyl column chromatography, these PH20 variants were eluted earlier than WT, suggesting that these variants all have a hydrophilic nature. However, the pattern of the main peak at the amino acid substitution position appeared differently (FIG. 5D).

The variant HM7 also showed two peaks in the phenyl column chromatography, like the variants HM1 and HM6, suggesting that two different types were present.

In order to examine the patterns of migration of WT and the variants depending on their isoelectric points, isoelectric focusing (hereinafter referred to as IEF) analysis was performed (FIG. 5E). On IEF gel, WT and the variant HM6 and HM8 showed similar migration patterns, and the variants HM9, HM10, HM21 and HM7 comprising the S343E mutation migrated to a more acidic region. This result suggests that a change in the isoelectric point of the protein occurs due to the introduction of glutamic acid by S344E substitution of the amino acid between G340 and I344.

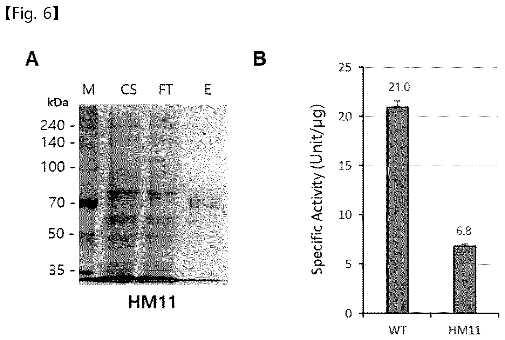

In addition, based on the variant HM6, other amino acids in the C-terminal regions of alpha-helix 8 were substituted, thereby constructing the variants HM11 and HM12. The sequences of the substituted amino acids in the variants HM11 and HM12 are shown in Table 11 above. The variant HM11 was expressed in ExpiCHO cells, but the expression level thereof was lower than that of WT (FIG. 2), and the variant HM12 was not expressed in ExpiCHO cells. The variant HM11 exhibited an activity corresponding to 32% of that of WT (FIG. 6B).

### Working Example 4. Construction of N-terminal amino acid-truncated PH20 variants based on HM6

The C-terminal region of PH20 is already well known to play an important role in the expression and enzymatic activity of PH20, but the role of the N-terminal region of PH20 is not well known. In order to examine the effect of truncation of the amino acid at the N-terminal region of PH20 on the enzymatic activity, variants HM40, HM13, HM41, HM24, HM42 and HM25 having the N-terminus truncated at N37, F38, R39, A40, P41 or P42 were constructed based on the variant HM6 (Table 11). Furthermore, HP61 and HP62 with modifications at the N-terminal amino acids were additionally constructed.

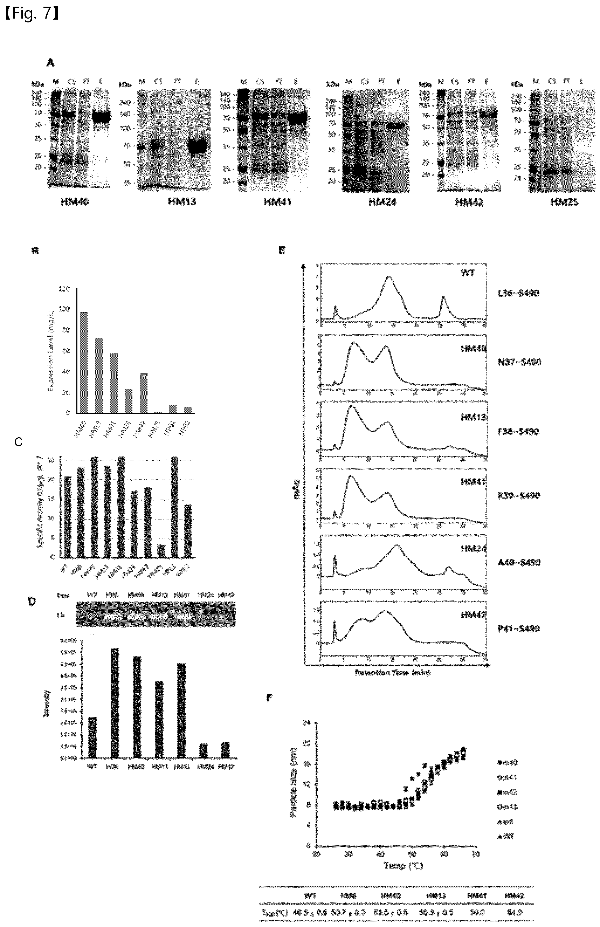

The variants HM40, HM13, HM41, HM24, HM42, HP61 and HP62 were expressed in ExpiCHO cells, but HM25 was not expressed (FIGS. 7A and 7B). The N-terminal truncated PH20 variants showed a difference in enzyme activity depending on the position where the N-terminus started. The variants HM40, HM13 and HM41, in which one to three amino acids were truncated, showed enzymatic activity similar to that of the template HM6, but HM24 and HM42, in which four to five amino acids were truncated, showed slightly lower activity than HM6 (FIG. 7C). However, HM25 in which six amino acids were truncated were little expressed in ExpiCHO cells, and the enzymatic activity was also significantly low (3.5 U/µg). It appears that changes in the enzymatic activities of HP61 and HP62 with modifications at N-terminal amino acids are not significant.

Regarding the enzymatic activities of the N-terminal truncated PH20 variants, measured by the substrate-gel assay (1 h of reaction), the enzymatic activities of HM40, HM13 and HM41 were similar to that of the template HM6, but the enzymatic activities of HM24 and HM42 were lower than that of HM6 (FIG. 7D). HM25 in which six amino acids were truncated could not be analyzed, because the amount of the protein produced was small.

When the physical properties of the variants HM40, HM13, HM41, HM24 and HM42 were analyzed using a phenyl column chromatography, these variants were eluted from the column earlier than WT, suggesting that these variants have a hydrophilic nature (FIG. 7E). This result suggests that the variants HM40, HM13, HM41, HM24 and HM42 constructed based on the variant HM6 maintained the hydrophilic nature of HM6 when considering the characteristics of L36 to A40 residues.

The aggregation temperatures of the N-terminal truncated PH20 variants, measured by DLS, were different among the variants depending on the position where the amino acid started (FIG. 7F). Although the N-terminal amino acid residues of the variants HM40, HM13, HM41 and HM42 were truncated, they showed an aggregation temperature of 50°C or higher, indicating that the characteristics of the template HM6 were remained intact. Among these variants, the variants HM40 and HM42 exhibited an aggregation temperature which was 3 to 4°C higher than that of HM6, indicating that the thermal stability of these variants increased. In addition, in order to examine the effect of substitution of the N-terminal amino acids of PH20 on protein expression and enzymatic activity, the following variants were constructed: the variant HM17 in which N-terminal amino acid sequences 36 to 47 (LNFRAPPVIPNV) of PH20 were substituted with FRGPLLPNR; and the variant HM18 in which N-terminal amino acid sequences 36 to 52 (LNFRAPPVIPNVPFLWA) of PH20 were substituted with FRGPLLPNRPFTTV. The sequences of the substituted amino acids in the variants HM17 and HM18 are shown in Table 11 above. The variants HM17 and HM18 were not expressed in ExpiCHO cells. This suggests that, even when up to five amino acids located at the N-terminus were truncated, the variants showed protein expression and enzymatic activity; however, substitution of more amino acid sequences, such as 36 to 47 sequences or 36 to 52 sequences, had an effect on protein refolding.

### Working Example 5. Construction of the C-terminal amino acid-truncated HM6-based variants HM14, HM15 and HM16 of PH20

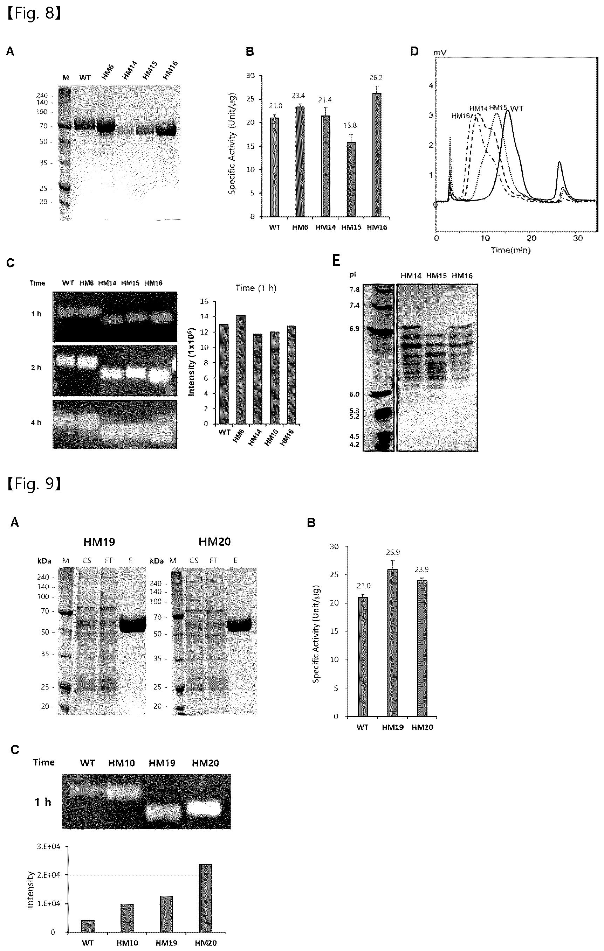

The C-terminal region of PH20 is known to play an important role in protein expression and enzymatic activity. In the present invention, the variants HM14, HM15 and HM16, in which the C-terminal amino acids were truncated at I465, F468 and K471, respectively, were constructed based on the variant HM6. The sequences of the substituted amino acids in these variants HM14, HM15 and HM16 are shown in Table 11 above. These variants HM14, HM15 and HM16 were expressed in ExpiCHO cells (FIG. 8A), and the protein expression levels thereof were in the order of HM16 > HM15 > HM14, indicating that the protein expression levels decreased as the number of C-terminal amino acids truncated increased (FIG. 8A). However, the enzymatic activities of the variants HM14, HM15 and HM16 were in the order of HM16 > HM14 (≡WT) > HM15 (FIG. 8B). According to Frost et al., the C-terminal 477-483 region of PH20 is necessary for soluble expression, and when the C-terminus is truncated at C467, the enzymatic activity of the variant is only 10% of a PH20 variant whose C-terminus was truncated at 477 to 483, and when the C-terminus is truncated before 467, the variant has no enzymatic activity. However, the C-terminal truncated variants HM14, HM15 and HM16 constructed based on the variant HM6 in the present invention showed increased protein refolding due to amino acid substitution of the M345 to I361 region, and thus the thermal stability thereof increased. For this reason, even when the C-terminus was truncated after 1465, F468 or P471, the variant showed enzymatic activity similar to that of WT, and the enzymatic activity thereof did not significantly decrease.

The structural flexibilities of WT and the variants HM14, HM15 and HM16 were examined by fluorescence quenching using acrylamide (FIG. 11B). The variants HM14, HM15 and HM16 were all structurally more flexible than WT. This result suggests that the C-terminal truncated variants constructed using the variant HM6 also maintained their structural flexibility.

The enzymatic activities of the variants HM14, HM15 and HM16, measured by turbidimetric assay, were also confirmed in substrate-gel assay (FIG. 8C).

When the physical properties of the C-terminal truncated variants were analyzed using a phenyl column chromatography, the variants HM14, HM15 and HM16 were all eluted earlier than WT, indicating that they had a hydrophilic nature. In addition, the hydrophilicities of these variants were in the order of HM16 > HM14 > HM15 (FIG. 8D).

### Working Example 6. Construction of the HM10-Based Variants HM19 and HM20 Comprising N-Terminal and C-Terminal Amino Acid Truncation

The PH20 variants constructed in the present invention were based on HM6, and HM8, HM9 and HM10, in which amino acid residues G340 to I344 were additionally substituted, exhibited better performance than WT in terms of protein expression levels, enzymatic activities and thermal stabilities. The variants HM19 and HM20 were constructed which had an N-terminus truncated at F38 and a C-terminus truncated at K470 or F468 based on HM10 having high enzymatic activity and thermal stability among variants HM8, HM9 and HM10. HM19 and HM20 were all expressed in ExpiCHO cells and purified using a HisTrap column chromatography (FIG. 9A). When the enzymatic activities of these variants were measured by turbidimetric assay, HM19 and HM20 exhibited an enzymatic activity which was 10% higher than that of WT (FIG. 9B). In substrate-gel assay, HM19 and HM20 also exhibited higher enzymatic activity than WT (FIG. 9C).

### Working Example 7. Characterization of HM10-Based PH20 Variants

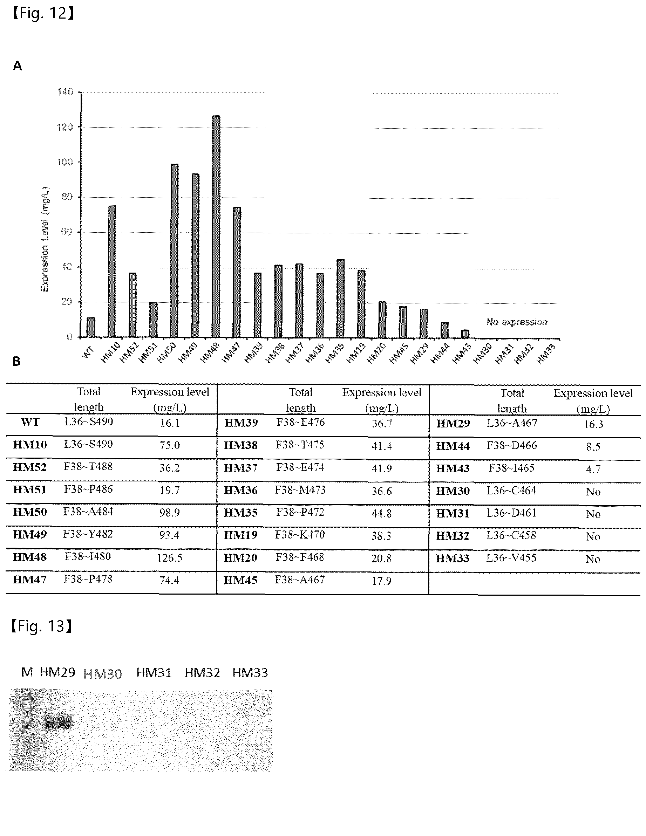

The expression levels of HM10-based C-terminal truncated variants in ExpiCHO cells showed a tendency to decrease as the length of the C-terminal region became shorter, and these variants were not expressed when the C-terminus was truncated at C464 or shorter (FIG. 12). C464 is necessary because it forms a disulfide bond with C437 and is important for maintaining the protein structure.

In order to examine whether the variant is not expressed in ExpiCHO cells when the C-terminus is truncated at 464 or shorter, Western blot analysis was performed. As shown in FIG. 13, the HM30, HM31, HM32 and HM33 variants were not detected in Western blots.

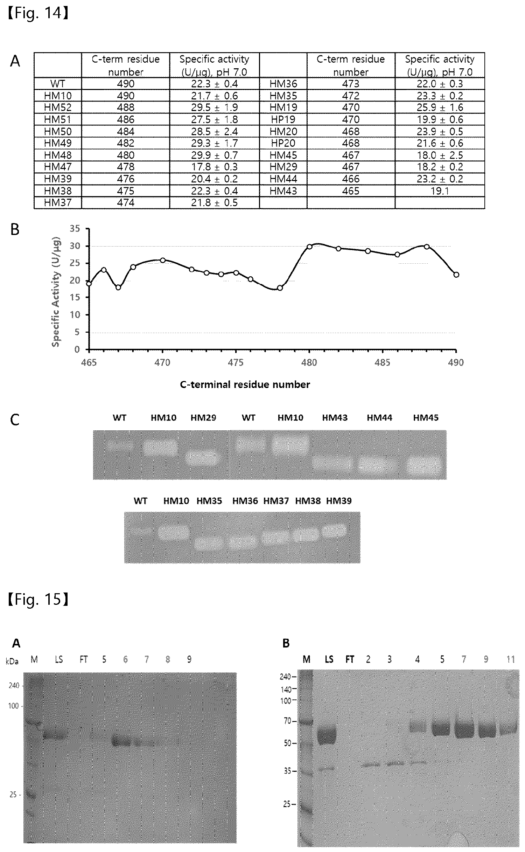

The enzymatic activities of the HM10-based C-terminal truncated variants, measured by turbidimetric assay, are shown in FIGS. 14A and 14B. The C-terminal truncated PH20 variants exhibited an enzymatic activity of ±20% compared to WT. When the C-terminus was truncated after I480 or beyond, the enzyme activity increased overall. In addition, the variants HP19 and HP20, obtained by removing the 6xHis-tag from HM19 and HM20, showed decreases in enzymatic activity of 23% and 9.6%, respectively, compared to when the 6xHis-tag was present. This suggests that the 6His-tag has an effect on the enzymatic activity.

When the enzymatic activities of the HM10-based C-terminal truncated variants were measured by substrate-gel assay, these variants exhibited higher enzymatic activity than WT and showed enzymatic activity similar to that of the template HM10, indicating that the difference in enzymatic activity depending on the length of the C-terminal region was not significant (FIG. 14C).

### Working Example 8. Characterization of HM21-Based PH20 Variants

The variant HP34 was purified by four-step column chromatography (FIG. 15A), and the variant HP46 was purified by three-step column chromatography (FIG. 15B). The amounts of HP34 and HP46 produced were 1.73 mg/L and 25.6 mg/L, respectively. HP34 and HP46 are 6xHis-tag-free variants, and the process of purifying these variants differs from the process of purifying variants having the 6xHis-tag, and thus it is difficult to compare the expression levels of the proteins.

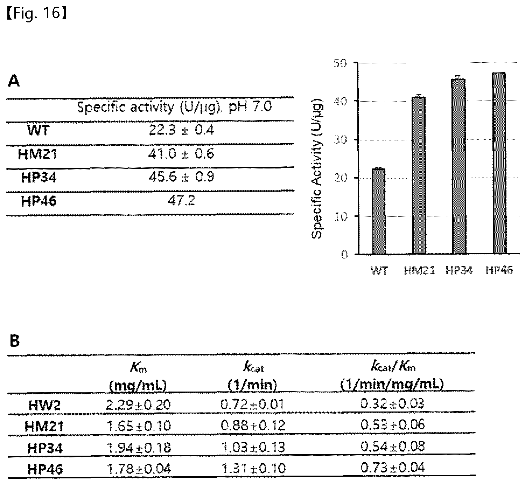

In turbidimetric assay, the activities of HP34 and HP46 were 45.6 U/µg and 47.2 U/µg, respectively, which were about 2-fold higher than that of WT and were about 10% higher than that of the template HM21 (FIG. 16A).

The kinetics of each variant was measured by Morgan-Elson assay, and the results of the measurement are shown in FIG. 16B. The catalytic efficiencies (*k*_{cat}/*K*ₘ) of HP34 and HP46 were 1.7 to 2 times higher than that of wild-type HW2. This result is consistent with the result that the specific activity was higher than that of WT. The Michaelis constant (*K*ₘ) was lower in these variants than in HW2, indicating the substrate affinities of these variants increased. From these results, it can be concluded that the HM21, HP34 and HP46 variants bind strongly to the substrate and have the property of converting the substrate into a product with high efficiency. This property is attributable to the effect of substitution of T341 with serine. It can be predicted that substituting threonine located at position 341 with an amino acid, such as alanine, glycine, aspartic acid or the like, would influence the enzymatic activity.

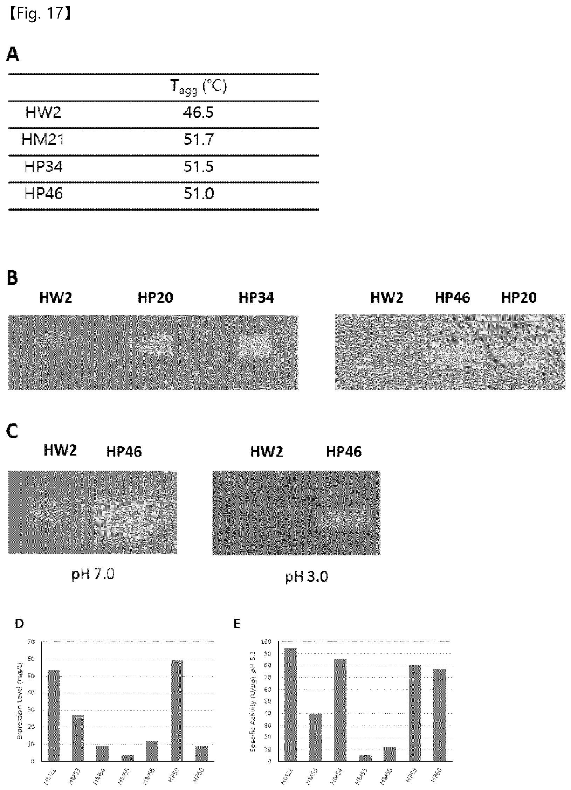

The aggregation temperatures of HP34 and HP46, measured by DLS, were 51.5°C and 51.0°C, respectively, which were similar to that of the template HM21 and were about 5°C higher than that of HW2, indicating that these variants were thermally stable (FIG. 17A). The enzymatic activity of HP34, measured by substrate-gel assay, was similar to that of HP20, whereas HP46 exhibited higher enzymatic activity than HP20, indicating that the protein refolding of HM21 as a template was better than that of HM10 (FIG. 17B).

Wild-type HW2 and the variant HP46 were left to stand overnight at pH 7.0 and pH 3.0, and then the enzymatic activities thereof were compared by substrate-gel assay. As a result, it was shown that HP46 exhibited high activity not only at pH 7.0 but also at pH 3.0, indicating that it had excellent stability (FIG. 17C).

HM53, HM54, HM55, HM56, HP59 and HP60 are variants having a mutation at the amino acid at position 341, among HM21-based variants. It was confirmed that the mutation of the amino acid at position 341 had various effects on the expression level and activity of the variants (FIGS. 17D and 17E)

### Working Example 9. In Vitro Immunogenicity Assay of PH20 Variant

Biopharmaceuticals with higher molecular weights than those of low-molecular-weight synthetic chemicals have a risk of triggering unintended immune responses when they enter the human body. An outside contact surface, created by refolding or interaction with adjacent domains in the secondary structure or tertiary structure of a large-molecular-weight biomaterial, can promote immune response to the biomaterial by providing an epitope to the immune system in the human body. This immune response can produce an anti-drug antibody (ADA), and this response can inhibit the drug's effectiveness, or cause hypersensitivity to the drug, or promote clearance of the drug in the human body. The immune response to the drug may therefore affect the results in clinical trials, and may cause serious abnormal reactions upon long-term use. This immune response can be influenced by various factors, and triggered by the specific response to the drug itself or disease, or by factors that depend on the method of drug administration or individual patients. Factors caused by the drug itself include the similarity or dissimilarity of the biopharmaceuticals to human peptides, posttranslational modification, impurities, aggregate formation, and the characteristics of the formulation. Factors that varies among individual patients include sex, reactivity with other drugs being taken, and genetic factors depending on the type of human leukocyte antigen (HLA).

This immunogenic response is triggered by CD4 + T cells or CD8 + T cells that recognize epitopes regardless of the cause of the immune response. Because of HLA class II gene diversity in individuals, epitopes of CD4 + T cells are different among individuals, and therefore the responsiveness to biopharmaceuticals in CD4+ T cells in each blood provided by the healthy donor can be a very important criterion for evaluating immune responses that may arise in clinical processes. CD4 + T cells are activated by antigen presenting cells (APCs) recognizing the antigens presented through their type II MHC (major histocompatibility complex). Activated CD4 + T cells release cytokines that activate macrophages, cytotoxic T cells, and B cells, resulting in high levels of antibody production. On the contrary, CD8 + T cells have direct cytotoxicity and directly remove cells infected with antigens, damaged cells, or cells that lost their function. CD8 + T cells have T cell receptors that can recognize specific antigen peptides bound to type I MHC molecules located on the surface of every cell. CD8 + T cells can also be activated by recognizing antigens presented by antigen presenting cells, and this activation can be further enhanced by cytokines of CD4 + T cells. Therefore, when the activation level of CD4 + T cells and CD8 + T cells by new biomaterials is measured *in vitro,* it is possible to predict immunogenic responses that can be induced in clinical processes. In this working example, in order to predict the immunogenicity of the PH20 variant in comparison with a control, CD4+ T cells and CD8+ T cells were isolated from PBMCs, and then treated with 1.5 ng/mL and 15 ng/mL of the control PH20 and the PH20 variant (HP46), and then the distributions of activated CD4+ T cells and CD8+ T cells were measured. The activation level of each type of T cells was measured using Stimulating Index, and Stimulating Index (SI) is defined as follows. If the SI value of cells is 2 or more, it can be determined that the cells were activated at the significant level.

Stimulating Index (SI) = (T-cell activation level after treatment with test sample) /

### (T-cell activation level after treatment with vehicle)

Immunogenic responses may vary depending on the HLA type. Therefore, experiments for measuring responses in more various HLA types were performed using T cells isolated from PBMCs provided from 10 healthy donors. The HLA types of the 10 PBMCs used are shown in Table 12 below.

**Table 12. HLA types of tested PBMCs**

| No. | HLA A | | HLA B | | HLA C1 | | DRB1 | | DQB1 | | DPB1 | |
|---|---|---|---|---|---|---|---|---|---|---|---|---|
| 1 | 0206 | 3303 | 3501 | 5801 | 0302 | 0801 | 1405 | 1501 | 0303 | 0602 | 0201 | 0301 |
| 2 | 0201 | 0207 | 4601 | 5101 | 0102 | 1502 | 0803 | 1101 | 0301 | 0601 | 0201 | 0202 |
| 3 | 0206 | 3101 | 1501 | 3501 | 0304 | 0401 | 0803 | 0901 | 0303 | 0601 | 0201 | 0501 |
| 4 | 0101 | 3101 | 1501 | 1517 | 0303 | 0701 | 1302 | 1501 | 0602 | 0604 | 0401 | 0501 |
| 5 | 0201 | 3101 | 5101 | 6701 | 0702 | 1402 | 0401 | 0403 | 0301 | 0302 | 0201 | 0401 |
| 6 | 0201 | 1101 | 1501 | 3901 | 0401 | 0702 | 0102 | 0901 | 0402 | 0501 | 0101 | 1401 |
| 7 | 0201 | 0206 | 4002 | 5502 | 0102 | 0304 | 0802 | 1454 | 0302 | 0502 | 0402 | 0501 |
| 8 | 0201 | 0201 | 1501 | 4001 | 0303 | 0304 | 0802 | 1406 | 0301 | 0302 | 0501 | 1301 |
| 9 | 1101 | 2402 | 4006 | 5101 | 0801 | 1502 | 0701 | 0901 | 0202 | 0303 | 0201 | 1301 |
| 10 | 2602 | 3101 | 4002 | 5101 | 0304 | 1502 | 0405 | 1501 | 0401 | 0602 | 0201 | 0501 |

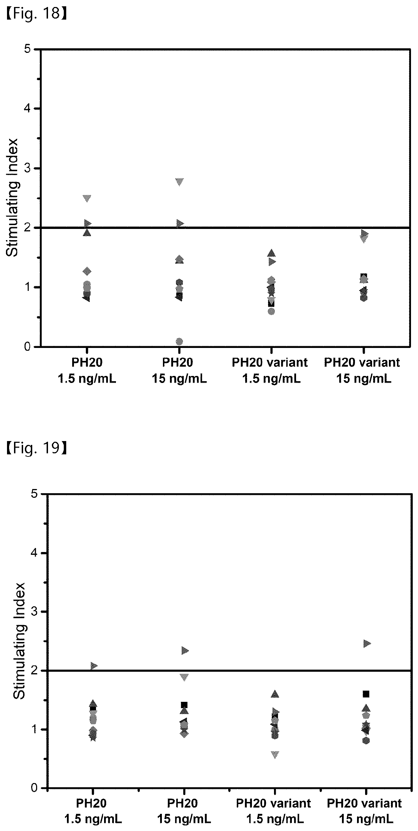

The results of measuring the activation levels of CD4+ and CD8+ T cells treated with each of PH20 and the PH20 variant are summarized in Table 13 below. When looking at the results of measuring the activation levels of CD4+ and CD8+ T cells, it appears that PH20 and the PH20 variant all show relatively low activation levels. In the case of PH20, the activation level of CD4+ T cells was measured to be 2 or more in two experiments, and in the case of the PH20 variant, the activation of CD4+ T cells was not detected. In the case of PH20, the activation of CD8+ T cells was detected in one experiment, and in the case of the PH20 variant, the activation of CD8+ T cells was also detected in one experiment. However, in the case of PH20, the SI value was measured to be 2 or more at both 1.5 ng/mL and 15 ng/mL, but in the case of the PH20 variant, an SI value of 2 or less at 1.5 ng/mL and an SI value of 2 or more at 15 ng/mL were measured (see FIGS. 18 and 19).

Therefore, at the lower concentration, the activation level of CD8 + T cells was observed to be low in the case of the PH20 variant, and it is determined that the activation level of CD8+ T cells by PH20 is higher than the activation level of CD8+ T cells by the PH20 variant. The conclusions drawn from the above results are as follows:
1) the activation levels of CD4+ T cells and CD8+ T cells by PH20 and the PH20 variant are relatively low; and
2) the possibility of activation of CD4+ T cells and CD8+ T cells by the PH20 variant is lower than that by PH20.

From these results, it is expected that the PH20 variant will have a lower possibility of triggering an immunogenic response in clinical processes than PH20.

**Table 13. Stimulating Index (SI) measured from in vitro immunogenicity assay results**

| PBMC No. | CD4 | | | | CD8 | | | |
|---|---|---|---|---|---|---|---|---|
| | PH20 (ng/mL) | | PH20 variant (ng/mL) | | PH20 (ng/mL) | | PH20 variant (ng/mL) | |
| | 1.5 | 15 | 1.5 | 15 | 1.5 | 15 | 1.5 | 15 |
| 1 | 1.03 | 0.859 | 0.728 | 1.176 | 1.38 | 1.42 | 1.22 | 1.60 |
| 2 | 1.05 | 0.09 | 0.596 | 0.922 | 1.20 | 1.11 | 1.00 | 1.07 |
| 3 | 1.90 | 1.44 | 1.56 | 1.12 | 1.43 | 1.31 | 1.59 | 1.35 |
| 4 | **2.51** | **2.79** | 0.788 | 1.82 | 1.28 | 1.90 | 0.579 | 0.974 |
| 5 | 1.27 | 1.47 | 1.09 | 1.14 | 0.987 | 0.932 | 0.972 | 1.02 |
| 6 | 0.825 | 0.834 | 0.998 | 0.946 | 0.904 | 1.13 | 1.09 | 0.986 |
| 7 | **2.07** | **2.07** | 1.43 | 1.90 | **2.08** | **2.34** | 1.30 | **2.46** |
| 8 | 0.898 | 1.079 | 0.967 | 0.822 | 0.926 | 1.06 | 0.896 | 0.813 |
| 9 | 0.882 | 0.957 | 0.895 | 0.941 | 0.853 | 0.993 | 0.950 | 1.09 |
| 10 | 0.983 | 0.970 | 1.12 | 1.13 | 1.15 | 1.08 | 1.15 | 1.24 |

### INDUSTRIAL APPLICABILITY

The PH20 variants or fragments thereof according to the present invention have increased protein expression levels and show an increase in protein aggregation temperature of 4-11.5°C or so when expressed in CHO (ExpiCHO) cells so that they can be efficiently produced while having high thermal stability, compared to the mature wild-type PH20.

Further, as the result of substrate-gel assay, one of tests to measure the activity of hyaluronidase, the PH20 variants or fragments thereof according to the present invention have improved protein refolding so that they are re-natured faster than that of the mature wild-type PH20, and the original enzymatic activity is maintained regardless of the C-terminal truncation position.

Furthermore, the PH20 variants or fragments thereof according to the present invention have low immunogenicity so that they can be repeatedly administered to the human body.

### References

Arming, S., Strobl, B., Wechselberger, C., and Kreil, G. (1997). In vitro mutagenesis of PH-20 hyaluronidase from human sperm. Eur J Biochem 247, 810-814.
Bookbinder, L.H., Hofer, A., Haller, M.F., Zepeda, M.L., Keller, G.A., Lim, J.E., Edgington, T.S., Shepard, H.M., Patton, J.S., and Frost, G.I. (2006). A recombinant human enzyme for enhanced interstitial transport of therapeutics. J Control Release 114, 230-241.
Chao, K.L., Muthukumar, L., and Herzberg, O. (2007). Structure of human hyaluronidase-1, a hyaluronan hydrolyzing enzyme involved in tumor growth and angiogenesis. Biochemistry 46, 6911-6920.
Frost, G.I. (2007). Recombinant human hyaluronidase (rHuPH20): an enabling platform for subcutaneous drug and fluid administration. Expert Opin Drug Deliv 4, 427-440.

### SEQUENCE LISTING (FREE TEXT)

The electronic file is attached.
For reasons of completeness, various aspects of the invention are set out in the following numbered embodiments:

### [Embodiment 1]

An PH20 variant or fragment thereof, which comprises one or more amino acid residue substitutions selected from the group consisting of T341A, T341C, T341G, S343E, M345T, K349E, L353A, L354I, N356E and I361T in wild-type PH20 having the amino acid sequence of SEQ ID NO: 1.

### [Embodiment 2]

The PH20 variant or fragment thereof of embodiment 1, which comprises one or more amino acid residue substitutions selected from the group consisting of T341A, T341C, L354I and N356E.

### [Embodiment 3]

The PH20 variant or fragment thereof of embodiment 1, which further comprises one or more amino acid residue substitutions in the region corresponding to an alpha-helix region and/or its linker region in wild-type PH20 having the amino acid sequence of SEQ ID NO: 1.

### [Embodiment 4]

The PH20 variant or fragment thereof of embodiment 3, wherein the alpha-helix region is an alpha-helix 8 region (S347 to C381), and its linker region is a linker region (A333 to R346) between alpha-helix 7 and alpha-helix 8.

### [Embodiment 5]

The PH20 variant or fragment thereof of embodiment 4, wherein the region corresponding to the alpha-helix region and its linker region is T341 to N363, T341 to I361, L342 to I361, S343 to I361, I344 to 1361, M345 to I361, or M345 to N363.

### [Embodiment 6]

The PH20 variant or fragment thereof of embodiment 4, wherein the alpha-helix 8 region (S347 to C381) and/or the linker region (A333 to R346) between alpha-helix 7 and alpha-helix 8 are/is substituted with portion(s) of amino acid residues of the corresponding region of Hyal1.

### [Embodiment 7]

The PH20 variant or fragment thereof of embodiment 1, which comprises amino acid residue substitutions of L354I and/or N356E, and further comprises an amino acid residue substitution at one or more positions selected from the group consisting of T341, L342, S343, I344, M345, S347, M348, K349, L352, L353, D355, E359, I361 and N363.

### [Embodiment 8]

The PH20 variant or fragment thereof of embodiment 7, which comprises amino acid residue substitutions of L354I and/or N356E, and further comprises one or more amino acid residue substitutions selected from the group consisting of T341A, T341C, T341D, T341G, T341S, L342W, S343E, I344N, M345T, S347T, M348K, K349E, L352Q, L353A, D355K, E359D, I361T and N363G.

### [Embodiment 9]

The PH20 variant or fragment thereof of embodiment 7, which comprises M345T, S347T, M348K, K349E, L352Q, L353A, L354I, D355K, N356E, E359D and I361T.

### [Embodiment 10]

The PH20 variant or fragment thereof of embodiment 9, which further comprises one or more amino acid residue substitutions selected from the group consisting of T341A, T341C, T341D, T341G, T341S, L342W, S343E, I344N and N363G.

### [Embodiment 11]

The PH20 variant or fragment thereof of embodiment 10, which comprises any one amino acid substitution selected from the group consisting of the following:
(a) T341S, L342W, S343E, I344N, M345T, S347T, M348K, K349E, L352Q, L353A, L354I, D355K, N356E, E359D and I361T;
(b) L342W, S343E, I344N, M345T, S347T, M348K, K349E, L352Q, L353A, L354I, D355K, N356E, E359D and I361T;
(c) M345T, S347T, M348K, K349E, L352Q, L353A, L354I, D355K, N356E, E359D, I361T and N363G;
(d) T341G, L342W, S343E, I344N, M345T, S347T, M348K, K349E, L352Q, L353A, L354I, D355K, N356E, E359D and I361T;
(e) T341A, L342W, S343E, I344N, M345T, S347T, M348K, K349E, L352Q, L353A, L354I, D355K, N356E, E359D and I361T;
(f) T341C, L342W, S343E, I344N, M345T, S347T, M348K, K349E, L352Q, L353A, L354I, D355K, N356E, E359D and I361T;
(g) T341D, L342W, S343E, I344N, M345T, S347T, M348K, K349E, L352Q, L353A, L354I, D355K, N356E, E359D and I361T;
(h) I344N, M345T, S347T, M348K, K349E, L352Q, L353A, L354I, D355K, N356E, E359D and I361T; and
(i) S343E, I344N, M345T, S347T, M348K, K349E, L352Q, L353A, L3541, D355K, N356E, E359D and I361T.

### [Embodiment 12]

The PH20 variant or fragment thereof of any one of embodiments 1 to 11, wherein portion(s) of the N-terminal or C-terminal amino acid residues are further deleted.

### [Embodiment 13]

The PH20 variant or fragment thereof of embodiment 12, wherein truncation occurs before an amino acid residue selected from the group consisting of M1 to P42 at the N-terminus, so that portion(s) of amino acid residues at the N-terminus are deleted.

### [Embodiment 14]

The PH20 variant or fragment thereof of embodiment 13, wherein truncation occurs before an amino acid residue L36, N37, F38, R39, A40, P41, or P42 at the N-terminus, so that portion(s) of amino acid residues at the N-terminus are deleted.

### [Embodiment 15]

The PH20 variant or fragment thereof of embodiment 12, wherein truncation occurs after an amino acid residue selected from the group consisting of V455 to L509 at the C-terminus, so that portion(s) of amino acid residues at the C-terminus are deleted.

### [Embodiment 16]

The PH20 variant or fragment thereof of embodiment 15, wherein truncation occurs after an amino acid residue selected from the group consisting of V455 to S490 at the C-terminus, so that portion(s) of amino acid residues at the C-terminus are deleted.

### [Embodiment 17]

The PH20 variant or fragment thereof of embodiment 16, wherein truncation occurs after an amino acid residue V455, C458, D461, C464, I465, D466, A467, F468, K470, P471, P472, M473, E474, T475, E476, P478, 1480, Y482, A484, P486, T488, or S490 at the C-terminus, so that portion(s) of amino acid residues at the C-terminus are deleted.

### [Embodiment 18]

The PH20 variant or fragment thereof of any one of embodiments 1 to 17, wherein the N-terminus comprises a human growth hormone-derived signal peptide having an amino acid sequence MATGSRTSLLLAFGLLCLPWLQEGSA of SEQ ID NO: 3, a human serum albumin-derived signal peptide having an amino acid sequence MKWVTFISLLFLFSSAYS of SEQ ID NO: 4, or a human Hyal1-derived signal peptide having an amino acid sequence MAAHLLPICALFLTLLDMAQG of SEQ ID NO: 5.

### [Embodiment 19]

The PH20 variant or fragment thereof of any one of embodiments 1 to 11, wherein the PH20 variant or fragment thereof is selected from the group consisting of the amino acid sequences of SEQ ID NOs: 60 to 115.

### [Embodiment 20]

The PH20 variant or fragment thereof of embodiment 19, which has the amino acid sequence of SEQ ID NO: 99.

### [Embodiment 21]

A composition for treating cancer, which comprises the PH20 variant or fragment thereof of any one of embodiments 1 to 20.

### [Embodiment 22]

The composition for treating cancer of embodiment 21, wherein the composition is used for combined treatment with other anticancer drugs.

### [Embodiment 23]

The composition for treating cancer of embodiment 22, wherein the other anticancer drugs are immuno-oncologic agents.

### [Embodiment 24]

The composition for treating cancer of embodiment 22, wherein the immuno-oncologic agents are immune checkpoint inhibitors.

### [Embodiment 25]

A nucleic acid encoding the PH20 variant or fragment thereof of any one of embodiments 1 to 20

### [Embodiment 26]

A recombinant expression vector comprising the nucleic acid of embodiment 25.

### [Embodiment 27]

A host cell transformed with the recombinant expression vector of embodiment 26.

### [Embodiment 28]

The host cell of embodiment 27, wherein the host cell is selected from the group consisting of animal cells, plant cells, yeasts, E. *coli.,* and insect cells.

### [Embodiment 29]

A method for producing a PH20 variant or fragment thereof, the method comprising a step of culturing the host cell of embodiment 28.

## Claims

1. A PH20 variant or fragment thereof, which comprises one or more amino acid residue substitutions selected from the group consisting of T341A, T341C, T341G, S343E, M345T, K349E, L353A, L354I, N356E and I361T in wild-type PH20 having the amino acid sequence of SEQ ID NO: 1.

2. The PH20 variant or fragment thereof of claim 1, which comprises one or more amino acid residue substitutions selected from the group consisting of T341A, T341C, L354I and N356E.

3. The PH20 variant or fragment thereof of claim 1, which further comprises one or more amino acid residue substitutions in the region corresponding to an alpha-helix region and/or its linker region in wild-type PH20 having the amino acid sequence of SEQ ID NO: 1,
optionally, wherein the alpha-helix region is an alpha-helix 8 region (S347 to C381), and its linker region is a linker region (A333 to R346) between alpha-helix 7 and alpha-helix 8,
further optionally wherein:
(i) the region corresponding to the alpha-helix region and its linker region is T341 to N363, T341 to I361, L342 to I361, S343 to I361, I344 to 1361, M345 to I361, or M345 to N363, or
(ii) the alpha-helix 8 region (S347 to C381) and/or the linker region (A333 to R346) between alpha-helix 7 and alpha-helix 8 are/is substituted with portion(s) of amino acid residues of the corresponding region of Hyal1.

4. The PH20 variant or fragment thereof of claim 1, which comprises amino acid residue substitutions of L354I and/or N356E, and further comprises an amino acid residue substitution at one or more positions selected from the group consisting of T341, L342, S343, 1344, M345, S347, M348, K349, L352, L353, D355, E359, I361 and N363, optionally wherein:
(i) the PH20 variant or fragment thereof comprises amino acid residue substitutions of L354I and/or N356E, and further comprises one or more amino acid residue substitutions selected from the group consisting of T341A, T341C, T341D, T341G, T341S, L342W, S343E, I344N, M345T, S347T, M348K, K349E, L352Q, L353A, D355K, E359D, I361T and N363G, and/or
(ii) the PH20 variant or fragment thereof comprises M345T, S347T, M348K, K349E, L352Q, L353A, L354I, D355K, N356E, E359D and I361T, optionally wherein the PH20 variant or fragment thereof further comprises one or more amino acid residue substitutions selected from the group consisting of T341A, T341C, T341D, T341G, T341S, L342W, S343E, I344N and N363G, further optionally wherein the PH20 variant or fragment thereof comprises any one amino acid substitution selected from the group consisting of the following:
(a) T341S, L342W, S343E, I344N, M345T, S347T, M348K, K349E, L352Q, L353A, L354I, D355K, N356E, E359D and I361T;
(b) L342W, S343E, I344N, M345T, S347T, M348K, K349E, L352Q, L353A, L354I, D355K, N356E, E359D and I361T;
(c) M345T, S347T, M348K, K349E, L352Q, L353A, L354I, D355K, N356E, E359D, I361T and N363G;
(d) T341G, L342W, S343E, I344N, M345T, S347T, M348K, K349E, L352Q, L353A, L354I, D355K, N356E, E359D and I361T;
(e) T341A, L342W, S343E, I344N, M345T, S347T, M348K, K349E, L352Q, L353A, L354I, D355K, N356E, E359D and I361T;
(f) T341C, L342W, S343E, I344N, M345T, S347T, M348K, K349E, L352Q, L353A, L354I, D355K, N356E, E359D and I361T;
(g) T341D, L342W, S343E, I344N, M345T, S347T, M348K, K349E, L352Q, L353A, L354I, D355K, N356E, E359D and I361T;
(h) I344N, M345T, S347T, M348K, K349E, L352Q, L353A, L354I, D355K, N356E, E359D and I361T; and
(i) S343E, I344N, M345T, S347T, M348K, K349E, L352Q, L353A, L354I, D355K, N356E, E359D and I361T.

5. The PH20 variant or fragment thereof of any one of claims 1 to 4, wherein portion(s) of the N-terminal or C-terminal amino acid residues are further deleted.

6. The PH20 variant or fragment thereof of claim 5, wherein truncation occurs before an amino acid residue selected from the group consisting of M1 to P42 at the N-terminus, so that portion(s) of amino acid residues at the N-terminus are deleted, optionally wherein truncation occurs before an amino acid residue L36, N37, F38, R39, A40, P41, or P42 at the N-terminus, so that portion(s) of amino acid residues at the N-terminus are deleted.

7. The PH20 variant or fragment thereof of claim 5, wherein truncation occurs after an amino acid residue selected from the group consisting of V455 to L509 at the C-terminus, so that portion(s) of amino acid residues at the C-terminus are deleted,
optionally wherein truncation occurs after an amino acid residue selected from the group consisting of V455 to S490 at the C-terminus, so that portion(s) of amino acid residues at the C-terminus are deleted,
further optionally wherein truncation occurs after an amino acid residue V455, C458, D461, C464, 1465, D466, A467, F468, K470, P471, P472, M473, E474, T475, E476, P478, 1480, Y482, A484, P486, T488, or S490 at the C-terminus, so that portion(s) of amino acid residues at the C-terminus are deleted.

8. The PH20 variant or fragment thereof of any one of claims 1 to 7, wherein the N-terminus comprises a human growth hormone-derived signal peptide having an amino acid sequence MATGSRTSLLLAFGLLCLPWLQEGSA of SEQ ID NO: 3, a human serum albumin-derived signal peptide having an amino acid sequence MKWVTFISLLFLFSSAYS of SEQ ID NO: 4, or a human Hyal1-derived signal peptide having an amino acid sequence MAAHLLPICALFLTLLDMAQG of SEQ ID NO: 5.

9. The PH20 variant or fragment thereof of any one of claims 1 to 4, wherein the PH20 variant or fragment thereof is selected from the group consisting of the amino acid sequences of SEQ ID NOs: 60 to 115, optionally wherein the PH20 variant or fragment thereof has the amino acid sequence of SEQ ID NO: 99.

10. A composition for treating cancer, which comprises the PH20 variant or fragment thereof of any one of claims 1 to 9,
optionally wherein the composition is used for combined treatment with other anticancer drugs,
further optionally wherein the other anticancer drugs are immuno-oncologic agents,
and further optionally wherein the immuno-oncologic agents are immune checkpoint inhibitors.

11. A nucleic acid encoding the PH20 variant or fragment thereof of any one of claims 1 to 9.

12. A recombinant expression vector comprising the nucleic acid of claim 11.

13. A host cell transformed with the recombinant expression vector of claim 12.

14. The host cell of claim 13, wherein the host cell is selected from the group consisting of animal cells, plant cells, yeasts, *E. coli.,* and insect cells.

15. A method for producing a PH20 variant or fragment thereof, the method comprising a step of culturing the host cell of claim 14.
